(19) Europäisches Patentamt / European Patent Office / Office européen des brevets

(11) **EP 4 575 980 B1**

(12) **EUROPEAN PATENT SPECIFICATION**

(45) Date of publication and mention of the grant of the patent:
**15.10.2025 Bulletin 2025/42**

(21) Application number: **23219886.1**

(22) Date of filing: **22.12.2023**

(51) International Patent Classification (IPC):
**G06T 5/50** $^{(2006.01)}$    **A61B 3/10** $^{(2006.01)}$

(52) Cooperative Patent Classification (CPC):
**G06T 5/50; A61B 3/0025; A61B 3/10; A61B 3/102;**
G06T 2207/10064; G06T 2207/10072;
G06T 2207/10116; G06T 2207/20081;
G06T 2207/20084; G06T 2207/20182;
G06T 2207/20216; G06T 2207/30041

(54) **NOISE REDUCTION IN RETINAL IMAGES**

RAUSCHVERMINDERUNG IN NETZHAUTBILDERN

RÉDUCTION DU BRUIT DANS DES IMAGES RÉTINIENNES

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(43) Date of publication of application:
**25.06.2025 Bulletin 2025/26**

(73) Proprietor: **Optos plc**
**Dunfermline, Scotland KY11 8GR (GB)**

(72) Inventor: **Clifton, David**
**Dunfermline, Scotland, KY11 8GR (GB)**

(74) Representative: **Hoffmann Eitle**
**Patent- und Rechtsanwälte PartmbB**
**Arabellastraße 30**
**81925 München (DE)**

(56) References cited:
EP-B1- 3 668 369       CN-B- 109 345 469
US-A1- 2019 130 170     US-B2- 10 383 516

• WANG XIANGHONG ET AL: "A two-step iteration mechanism for speckle reduction in optical coherence tomography", BIOMEDICAL SIGNAL PROCESSING AND CONTROL, ELSEVIER, AMSTERDAM, NL, vol. 43, 7 March 2018 (2018-03-07), pages 86 - 95, XP085388761, ISSN: 1746-8094, DOI: 10.1016/J.BSPC.2018.02.011

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

[Field]

Example aspects herein generally relate to techniques for suppressing noise in images of a retina of an eye and, more particularly, to the processing of a sequence of images of a region of a retina to generate a de-noised image of the region.

[Background]

Retinal imaging of various different modalities is widely used to detect pathological and age-related physiological changes in the retina of the eye. In all cases, successful detection of such changes requires retinal images to be in focus and to have as little noise and as few artifacts as possible. However, for patient safety, retinal imaging devices generally use low-power illumination sources, which can result in a lower signal-to-noise ratio (SNR) than might be achieved in the imaging other tissues. Furthermore, noise is inherent in some imaging techniques. For example, optical coherence tomography (OCT) images tend to contain speckle noise, which can reduce contrast and make it difficult to identify boundaries between strongly scattering structures. Fundus autofluorescence (FAF) images are derived from detections of low luminance level fluorescence, and often have a poor SNR owing to a combination of photon noise and amplifier (readout) noise.

Noise may be suppressed by adapting the image capture process, for example by using an averaging technique whereby multiple image frames are recorded and registered with respect to each other, with the registered images then being averaged to produce a de-noised image in which the noise from the component images averages out to a lower level. Noise may also be suppressed by post-capture image processing techniques, for example by using a convolutional neural network (CNN) or other supervised machine learning algorithm, which has been trained using averaged (de-noised) images to remove noise from a captured retinal image. However, both these approaches tend to result in valuable low-contrast anatomical features, including texture associated with the retina, being suppressed or absent in the final de-noised image. It would therefore be desirable to provide techniques for producing de-noised retinal images that retain the texture associated with the retina.

Further background is provided in the following documents.

US 2019/130170 A1 discloses an image processing apparatus comprising an information generation unit configured to generate motion contrast information about substantially a same region of an eye to be examined by using a plurality of pieces of tomographic information obtained by imaging substantially the same region at a predetermined time interval, and an information acquisition unit configured to obtain information about a change in a motion contract value of at least part of substantially the same region by using a plurality of pieces of motion contrast information at different times. A method for generating an OCT angiography (OCTA) image is described with reference to Figure 4 of US 2019/130170 A1, wherein an image similarity is calculated among m frames of similar tomographic images at the same position. Any one of m frames of tomographic images is selected as a template, and correlation values are calculated with the remaining (m-1) frames. Highly correlated images, of which the calculated correlation values are greater than or equal to a certain threshold, are selected. The threshold is set so that a frame or frames of low image correlation due to the subject's blinking or involuntary eye movement during fixation can be excluded. As a result of the image selection, the m frames of images obtained at the same position are sorted out as appropriate to select q frames of images. The possible range of q is $1 \leq q \leq m$. The selected q frames are then registered. Next, each frame is collated with the template to determine a position shift amount ($\delta x$, $\delta y$, $\delta \theta$). Specifically, normalized cross-correlation (NCC) is determined while changing the position and angle of the template. A difference in image position when NCC becomes maximum is determined as the position shift amount. Next, the signal position correction is applied to the (q-1) frames other than the template according to the position shift amount ($\delta x$, $\delta y$, $\delta \theta$), whereby frame registration is performed. The registered luminance images are then averaged to generate an average luminance image.

CN 109 345 469 A discloses a speckle denoising method in OCT imaging based on a conditional generation antagonism network, which comprises: acquiring a training image, preprocessing the training image, data amplification, model training and model use. The described scheme adopts a conditional generation antagonism network (Cgan) structure, obtains a mapping model from an OCT image containing speckle noise to an OCT image without noise through training, and then adopts the mapping model to eliminate speckle noise of a retinal OCT image. The scheme introduces the constraint condition of keeping edge details to train in the conditional generation antagonistic network structure, and obtains the OCT image speckle denoising model sensitive to edge information, so that the speckle denoising model can effectively remove speckle noise and better retain image detail information at the same time.

EP 3 668 369 B1 discloses a method of processing a sequence of images of a retina acquired by an ophthalmic device to generate retinal position tracking information that is indicative of a movement of the retina during the acquisition of the sequence of images. The method comprises: (i) receiving one or more images of the retina; (ii) calculating a cross-correlation between a reference image and an image based on the one or more received images to acquire an offset between the image and the reference image; and repeating processes (i) and (ii) to acquire, as the retinal position tracking information, respective offsets for the images that are based on the respective one or more received images. The method further comprises modifying the reference image while processes (i) and (ii) are being repeated, by determining a measure of similarity between correspondingly located regions of pixels in two or more of the received images and accentuating features in the reference image representing structures of the imaged retina in relation to other features in the reference image based on the determined measure of similarity.

[Summary]

The present invention provides a computer-implemented method according to Claim 1, a computer-implemented method according to Claim 12, a computer program according to Claim 13, a data processing apparatus according to Claim 14, and an ophthalmic imaging system according to Claim 15. Optional features are set out in the remaining claims.

[Brief Description of the Drawings]

Example embodiments will now be explained in detail, by way of non-limiting example only, with reference to the accompanying figures described below. Like reference numerals appearing in different ones of the figures can denote identical or functionally similar elements, unless indicated otherwise.

Figure 1 is a schematic illustration of an ophthalmic imaging system 100 comprising an ophthalmic imaging device 10 and a data processing apparatus 60 according to example embodiments herein.

Figure 2 is a schematic illustration of an example process by which the data processing apparatus 60 may processes a sequence of images 20 of a region of a retina of an eye to generate an averaged image 70 of the region.

Figure 3 is a schematic illustration of an example implementation of the data processing apparatus 60 in programmable signal processing hardware 300.

Figure 4 is a flow diagram illustrating a method according to a first example embodiment, by which the data processing apparatus 60 processes a sequence of images of a region of a retina to generate an averaged image of the region.

Figure 5 shows an example of a FAF image, and a 1.3-pixel displacement at an edge of the image caused by a 0.3-degree rotation about the centre of the image.

Figure 6A shows an example of a FAF image.

Figure 6B shows an averaged FAF image, which has been obtained by averaging the FAF image shown in Figure 6A with another FAF image which is rotationally offset from the image of Figure 6A by 4.5 degrees.

Figure 6C shows an averaged FAF image, which has been obtained by averaging the FAF image shown in Figure 6A with another FAF image which has a negligible rotational offset relative to the image of Figure 6A.

Figure 7 is a schematic illustration of a segmentation of a second sequence of images 15 to generate the sequence of images 20.

Figure 8 is a flow diagram illustrating a method according to a second example embodiment, by which the data processing apparatus 60 processes a sequence of images of a region of a retina to generate an averaged image of the region.

Figure 9A shows an example FAF image acquired by the ophthalmic imaging device 10.

Figure 9B shows an example of the averaged image 70 obtained by averaging 10 FAF images, including the FAF image of Figure 9A, that have been selected from a sequence of FAF images in accordance with the method of the second example embodiment.

Figure 10 is a flow diagram illustrating a method according to a third example embodiment, by which the data processing apparatus 60 trains a machine learning algorithm to filter noise from retinal images.

Figure 11 is a flow diagram illustrating the method by which the processor 320 generates each of the averaged retinal images from the respective sequence of retinal images in the third example embodiment.

[Detailed Description of Example Embodiments]

[First Example Embodiment]

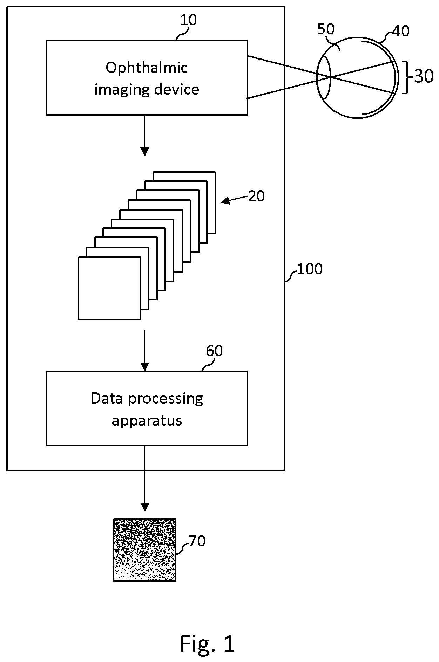

Figure 1 is a schematic illustration of an ophthalmic imaging system 100 according to an example embodiment, which comprises an ophthalmic imaging device 10, which is arranged to acquire a sequence of images 20 of a common region 30 of a retina 40 of an eye 50. The ophthalmic imaging system 100 further comprises a data processing apparatus 60, which is arranged to process images from the sequence of images 20 to generate an averaged image 70 of the region 30 of the retina 40.

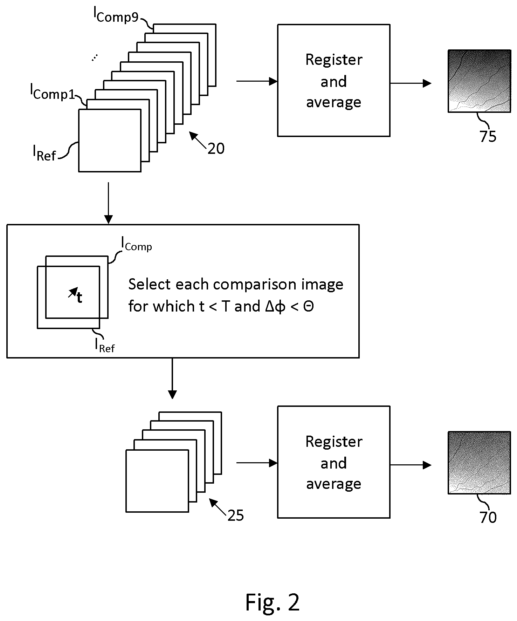

As illustrated schematically in Figure 2, the data processing apparatus 60 is arranged to generate the averaged image 70 by firstly selecting images from the sequence of images 20 according to one or more predefined criteria. These images are selected by firstly determining, for each combination of a reference image, $I_{Ref}$, which has been selected from the sequence of images 20 and a respective image from the sequence 20 being considered for the selection (herein referred to as a comparison image, $I_{Comp}$), a respective offset between the reference image $I_{Ref}$ and the respective comparison image $I_{Comp}$. Each determined offset is then compared with an offset threshold to determine whether the offset is smaller than the offset threshold. Specifically, the one or more predefined criteria include a criterion that the magnitude $t = |t|$ of a determined translational offset $t$ between the comparison image $I_{Comp}$ and the reference image $I_{Ref}$ is less that a predetermined translational offset threshold $T$. Additionally, the predefined criteria may include a criterion that the magnitude of a determined rotational offset (i.e. a relative rotation) $\Delta\phi$ between the comparison image $I_{Comp}$ and the reference image $I_{Ref}$ is less than a predetermined rotational offset threshold $\Theta$. The selected comparison images 25 are registered with respect to one another by the data processing apparatus 60, and the data processing apparatus 60 then calculates an average of the registered images, such that the respective pixel value of each pixel in the resulting averaged image 70 is a mean of the pixel values of correspondingly located pixels in the registered selected images 25 that correspond to respective common location in the region 30 of the retina 40.

In this process (examples of which are described below), in a case where the sequence of images 20 comprises at least one image which is offset from the reference image $I_{Ref}$ by an offset greater than the threshold, and images that are offset from the reference image $I_{Ref}$ by respective offsets that are smaller than the threshold, the one or more predefined criteria result in images being selected in such a way that the resulting averaged image 70 shows more texture than a reference averaged image 75 generated from some or all the images in the sequence of images 20, without these images having been selecting in the way herein described. In this context, the texture is an anatomical texture associated with the retina, which is indicative of an anatomical structure in the region 30 of the retina 40. For example, where the images acquired by the ophthalmic imaging device 10 are fundus autofluorescence (FAF) images, as in the present example embodiment, the structure may be defined by a spatial distribution of fluorophores across the region 30 of the retina 40. As another example, where the images acquired by the ophthalmic imaging device 10 are OCT images, the structure may comprise a physical structure of one or more layers of the retina 40 in the region 30 of the retina 40. As a further example, where the images acquired by the ophthalmic imaging device 10 are reflectance images (e.g. red, blue or green reflectance images) of the region 30 of the retina 40, the structure may comprise the upper surface of the retina in the region 30, such that the texture is a physical texture of the surface that reflects the topography of the surface.

Furthermore, the averaged image 70 tends to have a higher SNR than a single image from the sequence of images 20, owing to the noise components in the selected images 25 partially cancelling each other out because of the selected images 25 being averaged. The averaged image 70 generated by the data processing apparatus 60 of the example embodiment is thus a de-noised image of the region 30 of the retina 40, in which more of the texture is retained than in a conventional de-noised image (e.g. image 75) which has been generated by averaging images of the sequence 20 that have not been selected according to one or more of the criteria described herein. The image processing techniques described herein may thus produce de-noised and anatomically correct retinal images, in which important clinical data is preserved. Such de-noised images are valuable not only to clinicians for assessing the health of the retina 40 but also for the training of artificial intelligence (AI) denoising algorithms to suppress noise whilst reducing or avoiding a loss of texture in the de-noised image that often results from the use of conventionally trained AI denoising algorithms, as discussed in more detail below.

Suppression of noise whilst retaining texture in the averaged image 70 requires the processing of the sequence of images 20 to take into account the following classes of imperfections in the image acquisition process and their effects on the acquired images. Firstly, the reference image $I_{Ref}$ and a comparison image $I_{Comp}$ may be related by an affine or a non-affine transformation (e.g. a translation, a rotation or a warping in one or more directions), which may be caused by a movement of the retina during acquisition of the images which is in a direction normal to the imaging axis, a rotation of the retina about the imaging axis or a movement of the retina which occurs as a result of a change in gaze direction of the eye.

Secondly, an acquired image may have a non-affine warping within it, which may be caused by an eye movement, such as a rapid horizontal saccade, which occurs during capture of the image. Thirdly, there may be one or more localised regions or patches of micro-warping (micro-misalignment) within some of the images, which may be caused by variations in the imaging system (e.g. a beam scanning system therein comprising a polygonal mirror or other scanning element) and consequent light path variations between captures of different images. The light path variation may, for example, be caused by deviations from scan linearity and/or mirror imperfections, among other factors.

The ophthalmic imaging device 10 may, as in the present example embodiment, be any kind of FAF imaging device well-known to those versed in the art (e.g. a fundus camera, a confocal scanning laser ophthalmoscope (SLO) or an ultra-widefield imaging device such as the Daytona™ from Optos™), which is arranged to capture a sequence of FAF images of the same region of the retina 30. FAF imaging devices often use short to medium wavelength visible excitation, and collect emissions within a predefined spectral band (e.g. within a wavelength range of 500 to 750 nm) to form a brightness map reflecting the distribution of lipofuscin, which is a dominant fluorophore located in the retinal pigment epithelium (RPE). However, other excitation wavelengths, such as near-infrared, can be used to detect additional fluorophores, such as melanin. FAF is useful in the evaluation of a variety of diseases involving the retina and RPE.

The form of the ophthalmic imaging device 10 is not so limited, however. In other example embodiments, the ophthalmic imaging device 10 may, for example, take the form of an OCT imaging device, such as a swept-source OCT (SS-OCT) imaging device or a spectral domain OCT (SD-OCT) imaging device, which is arranged to acquire OCT images, e.g. B-scans, C-scans and/or en-face OCT images, of the region 30 of the retina 40. In yet other example embodiments, the ophthalmic imaging device 10 may comprise an SLO, a fundus camera or the like, which is arranged to capture reflectance images (e.g. red, green or blue images) of the region 30 of the retina 40.

Although the ophthalmic imaging device 10 is shown in Figure 1 to acquire a sequence of 10 images, this number has been given by way of an example only. The number of images in the sequence of images 20 is not limited to 10, and there may be more or fewer images in the sequence of images 20. It is also noted that the region 30 is a portion of the retina 40 which lies within a field of view (FoV) of the ophthalmic imaging device 10, and may be smaller than a portion of the retina 40 that is spanned by the FoV. As described in more detail below, the region 30 may be a segment of a larger region of the retina 40 which the ophthalmic imaging device 10 may be arranged to capture images of, where each image spans the field-of-view of the ophthalmic imaging device 10. Each image in the sequence of images 20 may therefore be a segment (e.g. a rectangular image tile) of a respective larger retinal image of the retina 40 which has been captured by the ophthalmic imaging device 10.

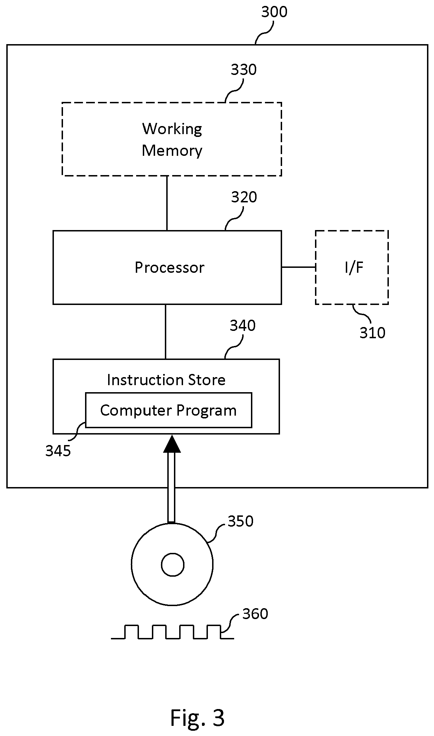

The data processing apparatus 60 may be provided in any suitable form, for example as a programmable signal processing hardware 300 of the kind illustrated schematically in Figure 3. The programmable signal processing apparatus 300 comprises a communication interface (I/F) 310, for receiving the sequence of images 20 from the ophthalmic imaging device 10, and outputting the averaged image 70. The signal processing hardware 300 further comprises a processor (e.g. a Central Processing Unit, CPU, and/or a Graphics Processing Unit, GPU) 320, a working memory 330 (e.g. a random-access memory) and an instruction store 340 storing a computer program 345 comprising the computer-readable instructions which, when executed by the processor 320, cause the processor 320 to perform the various functions of the data processing apparatus 60 described herein. The working memory 330 stores information used by the processor 320 during execution of the computer program 345, such as the reference image $I_{Ref}$, the comparison images $I_{Comp1}$ to $I_{Comp9}$, the calculated image offsets, image offset thresholds, selected comparison images 25, determined degrees of similarity, the similarity thresholds and the various intermediate processing results described herein. The instruction store 340 may comprise a ROM (e.g. in the form of an electrically erasable programmable read-only memory (EEPROM) or flash memory) which is pre-loaded with the computer-readable instructions. Alternatively, the instruction store 340 may comprise a RAM or similar type of memory, and the computer-readable instructions of the computer program 345 can be input thereto from a computer program product, such as a non-transitory, computer-readable storage medium 350 in the form of a CD-ROM, DVDROM, etc. or a computer-readable signal 360 carrying the computer-readable instructions. In any case, the computer program 345, when executed by the processor 320, causes the processor 320 to perform the functions of the data processing apparatus 60 described herein. Thus, the data processing apparatus 60 of the example embodiment may comprise a computer processor 320 (or two or more such processors) and a memory 340 (or two or more such memories) storing computer-readable instructions which, when executed by the processor(s), cause the processor(s) to process the sequence of images 20 to generate the averaged image 70 of the region 30 of the retina 40 as herein described.

It should be noted, however, that the data processing apparatus 60 may alternatively be implemented in non-programmable hardware, such as an ASIC, an FPGA or other integrated circuit dedicated to performing the functions of the data processing apparatus 60 described herein, or a combination of such non-programmable hardware and programmable hardware as described above with reference to Figure 3. The data processing apparatus 60 may be provided as a stand-alone product or as part of the ophthalmic imaging system 100.

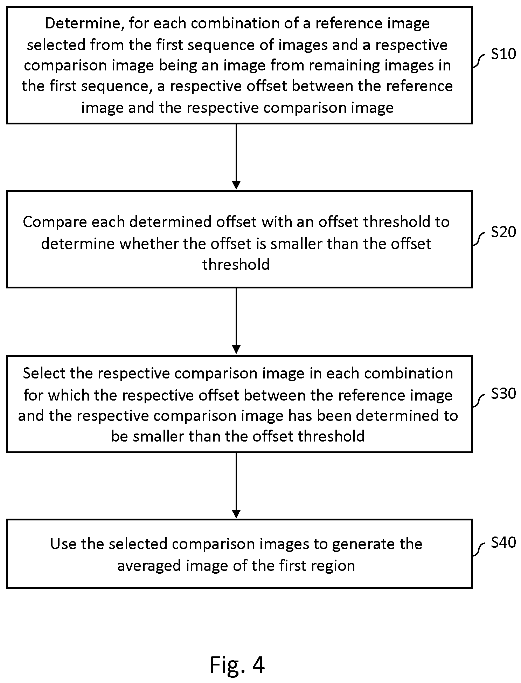

A process by which the data processing apparatus 60 of the present example embodiment processes the sequence of images 20 to generate the averaged image 70 of the region 30 of the retina 40 will now be described with reference to Figure 4.

In process S10 of Figure 4, the processor 320 of the data processing apparatus 60 determines, for each combination of a reference image $I_{Ref}$, which has been selected from the sequence of images 20, and a respective comparison image $I_{Comp}$ from remaining images in the sequence of images 20, a respective offset between the reference image $I_{Ref}$ and the respective comparison image $I_{Comp}$. The respective offset may comprise a rotation of the comparison image $I_{Comp}$ in the combination relative to the reference image $I_{Ref}$ in the combination and includes a translation of the comparison image $I_{Comp}$ in the combination relative to the reference image $I_{Ref}$ in the combination.

The reference image $I_{Ref}$ may be selected from the sequence of images 20 in a variety of different ways. The reference image $I_{Ref}$ may, for example, be the image that appears at a predetermined position in the sequence of images 20, for example at the beginning, at the end, or preferably at an intermediate position in the sequence of images 20 (e.g. the middle of the sequence). The reference image $I_{Ref}$ may alternatively be selected at random from the images in the sequence of images 20. However, to more reliably achieve as large a number of selected images 25 as possible, and thus make noise suppression in the averaged image 70 (which is derived from the selected images 25) more effective, the reference image $I_{Ref}$ is preferably selected to be an image from among the images in the sequence of images 20 for which the number of remaining images in the sequence that are offset from the reference image $I_{Ref}$ by an amount less than the predetermined offset threshold is highest (or equal highest). The reference image $I_{Ref}$ may, for example, be selected by determining, for any image chosen from the sequence of images 20 to serve as a base image, a respective translational offset between the base image and each image of the remaining images in the sequence of images 20, for example by determining the respective location of a peak in a cross-correlation calculated between the base image and each image of the remaining images. The translational offsets determined in this way may be represented as a set of points in a two-dimensional plot. For each point in this plot, the respective number of remaining points that are within a circle of radius T centred on the point is determined. Once this has been done for all the points in the plot, a point having the highest (or equal highest) number of remaining points within the circle centred on it is selected. The image from the sequence of images, whose offset from the base image is represented by the selected point, is then selected as the reference image $I_{Ref}$.

The processor 320 thus determines, for each image of the remaining images in the sequence 20 (i.e. other than the base image), a respective count by determining a respective translational offset between the image and every other image in the sequence, comparing the determined translational offsets with the offset threshold T to identify every other image in the sequence having a translational offset from the image which is smaller than the translational threshold T, and counting the identified images to determine the respective count. The processor 320 then uses the determined counts to identify, as the reference image $I_{Ref}$, an image of the remaining images in the sequence 20 for which the determined count is highest (or equal highest).

The processor 320 determines in process S10 of Figure 4, for each combination of the reference image $I_{Ref}$ and a respective comparison image $I_{Comp}$ from some or all the remaining images in the sequence of images 20, a respective translational offset between the reference image $I_{Ref}$ and the respective comparison image $I_{Comp}$, and a respective rotational offset between the reference image $I_{Ref}$ and the respective comparison image $I_{Comp}$. However, in other example embodiments, the processor 320 determines, for each combination of the reference image $I_{Ref}$ and a respective comparison image $I_{Comp}$ from remaining images in the sequence of images 20, a respective translational offset between the reference image $I_{Ref}$ and the respective comparison image $I_{Comp}$, and not a respective rotational offset between the reference image $I_{Ref}$ and the respective comparison image $I_{Comp}$.

The translational and rotational offsets between images may be determined using various techniques as described herein or well-known to those versed in the art. For example, in cases where relative rotations between images in the sequence of images 20 are negligible, the respective translational offset t between the reference image $I_{Ref}$ and each comparison image $I_{Comp}$ may be determined by calculating a cross-correlation between the reference image $I_{Ref}$ and the comparison image $I_{Comp}$, with the location of a peak in the calculated cross-correlation being used to determine the respective translational offset t. As the self-similarity of the images in the sequence of images 20 may result in relatively broad peaks in the calculated cross-correlations, the images may be pre-processed prior to being cross-correlated (using an edge detector, for example), to sharpen the peaks and thus allow the translational offset $t$ to be determined more precisely.

As an alternative, the respective translational offset t between the reference image $I_{Ref}$ and each comparison image $I_{Comp}$ may be determined by firstly calculating a normalized cross-power spectrum of the reference image $I_{Ref}$ and the comparison image $I_{Comp}$, which may be expressed as $F_{Ref}F_{Comp}^{*}/|F_{Ref}F_{Comp}^{*}|$, where $F_{Ref}$ is a discrete Fourier transform of the reference image $I_{Ref}$, and $F_{Comp}^{*}$ is the complex conjugate of a discrete Fourier transform of the comparison image $I_{Comp}$. An inverse Fourier transform of the normalized cross-power spectrum may then be calculated to provide a delta function which has a maximum value at $(\Delta x, \Delta y)$, where the comparison image $I_{Comp}$ is shifted by $\Delta x$ pixels in an x-axis direction relative to the reference image $I_{Ref}$, by $\Delta y$ pixels in a y-axis direction relative to the reference image $I_{Ref}$.

The cross-correlation approach described above may, as in the present example embodiment, be extended to allow for relative rotations between the images in the sequence of images 20 by determining a respective translational offset t between the reference image $I_{Ref}$ and each of a plurality of rotated versions of the comparison image $I_{Comp}$. This may be done by calculating a respective cross-correlation between the reference image $I_{Ref}$ and each rotated version of the comparison image $I_{Comp}$. By way of an example and without limitation, each comparison image $I_{Comp}$ (with or without the preprocessing mentioned above) may be rotated between -6 degrees and +6 degrees in increments of 0.1 degrees to generate 120 rotated versions of each comparison image $I_{Comp}$, each of which (along with the original (unrotated) comparison image $I_{Comp}$) is cross correlated with the reference image $I_{Ref}$. The angular range of -6 degrees to +6 degrees, and the increments of 0.1 degrees, have been given by way of example only, and other ranges and/or angular increments may alternatively be used. The rotation (if any) applied to the image that is cross-correlated with the reference image $I_{Ref}$ to produce a cross-correlation having the highest peak of the peaks in the 121 calculated cross-correlations may be taken to provide an indication of the rotational offset between the reference image $I_{Ref}$ and the comparison image $I_{Comp}$, while the location of the highest cross-correlation peak may be taken to provide an indication of the translational offset t between the reference image $I_{Ref}$ and the comparison image $I_{Comp}$.

The rotational offset $\Delta\phi$ and the translational offset t between the reference image $I_{Ref}$ and the comparison image $I_{Comp}$, may alternatively be calculated in the Fourier domain. This approach relies on two properties of the Fourier transform. First, the frequency spectrum is always centred on the origin, regardless of any shifts in the underlying image. Second, the rotation of an image will always result in a corresponding rotation in its Fourier transform. In the alternative approach, the reference image $I_{Ref}$ and the comparison image $I_{Comp}$ are transformed to polar coordinates. An image I(x, y) is thus transformed to $I(r, \vartheta)$, and the discrete Fourier transform of the image, F(u, v), becomes F($\rho$, $\phi$), so that

$$F_{Ref}(\rho, \phi) = \iint I_{Ref}(r, \theta) e^{-2\pi i \rho r \cos(\theta - \phi)} r \, dr \, d\theta$$

Given an angle $\beta$, such that $I_{Comp}$ is the rotation of $I_{Ref}$ by $\beta$ in the clockwise direction, it can be seen that $I_{Comp}(r, \vartheta) = I_{ref}(r, \vartheta + \beta)$. This means that

$$F_{Comp}(\rho, \phi) = \iint I_{Ref}(r, \theta + \beta) e^{-2\pi i \rho r \cos(\theta - \phi)} r \, dr \, d\theta$$

which is equivalent to

$$F_{Comp}(\rho, \phi) = \iint I_{Ref}(r, \theta) e^{-2\pi i \rho r \cos(\theta - (\phi + \beta))} r \, dr \, d\theta$$

so that

$$F_{Comp}(\rho, \phi) = F_{Ref}(\rho, \phi + \beta).$$

These two properties separate the rotational and translational components of image registration so that they can be solved independently. For a set of images that are both shifted and rotated with respect to each other, the rotational shift between the images can be isolated by taking their Fourier transform. The magnitude of the frequency components created by this Fourier transform are treated as if they are the pixel values for a new set of images that only require rotational registration. Once the rotational correction is found, it is applied to the underlying image to be registered. The translational parameters are then found using the same algorithm. The respective rotational offset $\Delta\phi$ between the reference image $I_{Ref}$ and the respective comparison image $I_{Comp}$ may thus be determined by calculating an inverse Fourier transform of a normalized cross-power spectrum of polar transformations of the reference image $I_{Ref}$ and the respective comparison image $I_{Comp}$. Further details of this approach are provided in the article titled "Medical Image Registration Using the Fourier Transform" by J. Luce et al., International Journal of Medical Physics, Clinical Engineering and Radiation Oncology, 2024, 3, pages 49-55 (February 2014), the contents of which are incorporated herein by reference in their entirety.

Other intensity-based algorithms may alternatively be used to determine offsets between the reference image $I_{Ref}$ and the comparison images $I_{Comp}$ in process S10 of Figure 4, including those based on similarity measures other than cross-correlation, for example mutual information, sum of squared intensity differences, or ratio image uniformity (among others).

Referring again to Figure 4, in process S20, the processor 320 compares each determined offset with an offset threshold to determine whether the offset is smaller than the offset threshold. The processor 320 compares each translational offset t with the translational offset threshold T to determine whether the translational offset t is smaller than the translational offset threshold T. The processor 320 may, as in the present example embodiment, also compare each rotational offset $\Delta\phi$ with the rotational offset threshold $\Theta$ to determine whether the rotational offset $\Delta\phi$ is smaller than the rotational offset threshold $\Theta$. In other example embodiments, where a respective translational offset between the reference image $I_{Ref}$ and the respective comparison image $I_{Comp}$, and not a respective rotational offset between the reference image $I_{Ref}$ and the respective comparison image $I_{Comp}$, is determined by the processor 320 for each combination of the reference image $I_{Ref}$ and a respective comparison image $I_{Comp}$ from remaining images in the sequence of images 20 (in process S10 of Figure 4), the processor 320 determines whether the translational offset t is smaller than the translational offset threshold T. The setting of the offset threshold values T and $\Theta$ is described below.

In process S30 of Figure 4, the processor 320 selects the respective comparison image $I_{Comp}$ in each combination for which the respective offset between the reference image $I_{Ref}$ and the respective comparison image $I_{Comp}$ has been determined to be smaller than the offset threshold. For any combination for which the respective offset between the reference image $I_{Ref}$ and the respective comparison image $I_{Comp}$ has been determined to not be smaller than the offset threshold, the processor 320 does not select the comparison image $I_{Comp}$ in that combination.

The processor 320 selects the respective comparison image $I_{Comp}$ in each combination for which the respective translational offset t has been determined to be smaller than the translational offset threshold T, and for which the respective rotational offset $\Delta\phi$ has been determined to be smaller than the rotational offset threshold $\Theta$ in process S20 of Figure 4. In other example embodiments, where a respective translational offset between the reference image $I_{Ref}$ and the respective comparison image $I_{Comp}$, and not a respective rotational offset between the reference image $I_{Ref}$ and the respective comparison image $I_{Comp}$, is determined by the processor 320 for each combination of the reference image $I_{Ref}$ and a respective comparison image $I_{Comp}$ from remaining images in the sequence of images 20 (in process S10 of Figure 4), the processor 320 selects the respective comparison image $I_{Comp}$ in each combination for which the respective translational offset t has been determined to be smaller than the translational offset threshold T.

In process S40 of Figure 4, the processor 320 uses the selected comparison images 25 to generate the averaged image 70 of the region 30 of the retina 40. The processor 320 uses the selected comparison images 25 (and optionally also the reference image $I_{Ref}$) to generate the averaged image 70 of the region 30 by registering the selected images 25 (and optionally also the reference image $I_{Ref}$) with respect to each other, and averaging the registered images to generate the averaged image 70 of the first region 30, such that the respective pixel value of each pixel in the averaged image 70 is a mean of the pixel values of correspondingly located pixels in the registered images that correspond to respective common location in the region 30 of the retina 40.

The offset thresholds T and $\Theta$ are selected such that, in case the sequence of images 20 comprises at least one image which is offset from the reference image $I_{Ref}$ by a translational offset t greater than the translational offset threshold T and optionally by a rotational offset $\Delta\phi$ greater than the rotational offset threshold $\Theta$, and images whose respective translational and rotational offsets from the reference image $I_{Ref}$ are smaller than T and $\Theta$, respectively, the averaged image 70 shows more texture which is indicative of a structure in the region 30 of the retina 40 than the reference averaged image 75 generated from the images in the sequence of images 20.

As the images being compared in process S20 of Figure 4 often differ by a non-affine transformation that tends to produce a deformation which increases with increasing offset (whether translational or rotational) between the images, a higher correlation between the content of the images, and therefore improved retention of texture in the averaged image 70, may be achieved by selecting comparison images $I_{Comp}$ that are offset from the reference image $I_{Ref}$ by less than the offset threshold. Any affine frame offsets (typically a translation and/or rotation) the selected images 25 may be effectively compensated for by the image registration performed in process S40 of Figure 4 such that the registered images are highly correlated.

The amount of texture in the averaged image 70 may be quantified using an algorithm as described in the article "Detection of Textured Areas in Images Using a Disorganization Indicator Based on Component Counts" by R. Bergman et al., J. Electronic Imaging. 17. 043003 (2008), the contents of which are incorporated herein by reference in their entirety. The texture detector presented in this article is based on the intuition that texture in a natural image is "disorganized". The measure used to detect texture examines the structure of local regions of the image. This structural approach allows both structured and unstructured texture at many scales to be detected. Furthermore, it distinguishes between edges and texture, and also between texture and noise. Automatic detection results are shown in the article to match human classification of corresponding image areas. The amounts of texture in the averaged image 70 and the reference averaged image 75 may be compared by comparing the areas of these images that are designated as 'texture' by the algorithm.

The inventors have found that the use of interpolation to register the selected images 25 with respect to each other in process S40 of Figure 4 tends to remove at least some of the texture that is present in the images acquired by the ophthalmic imaging device 10, and that more of the original texture may be retained in the averaged image 70 by registering the selected images 25 without interpolation, i.e. without interpolating between pixel values of any of the selected images 25 to register the images. The avoidance of interpolation in process S40 was found to not only improve the retention of this clinical data in the averaged image 70 but to also significantly improve the effectiveness of a machine learning (ML) denoising algorithm in distinguishing textural features from noise when the ML denoising algorithm has been trained using averaged images 70 that have been generated without interpolation.

Accordingly, the selected comparison images 25 are used by the processor 320 to generate the averaged image 70 of the region 30 by the processor 320 firstly registering the selected comparison images 25 with respect to one another. In this process, one of the selected images 25 may be selected as a base image, and each of the remaining selected images is registered with respect to the base image, in turn, to generate a set of registered images. Where an image of the remaining images is being registered with respect to the base image, pixel values of a first image of the images in this pair of images are redistributed according to a geometric transformation between image coordinate systems of the images in the pair, i.e. pixel values of the first image are maintained but reassigned to different pixels in the first image in accordance with the geometric transformation that is required to register the images so that no interpolation is performed. The processor 320 then generates the averaged image 70 of the region 30 by averaging the registered images, such that the respective pixel value of each pixel in the averaged image 70 is a mean of the pixel values of correspondingly located pixels in the registered selected images 25 that correspond to respective common location in the region 30 of the retina 40.

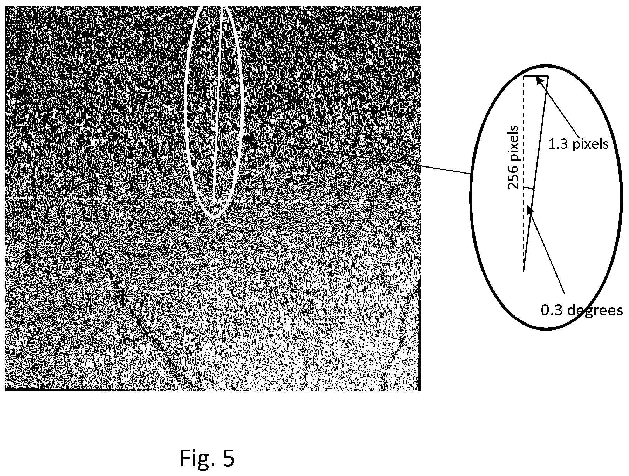

In case interpolation is avoided in process S40 of Figure 4, the respective geometric transformation between the image coordinate systems of the images in each pair of the selected comparison images 25 consists of (i) a respective first translation, by a respective first integer numbers of pixels, along a first pixel array direction along which pixels of the selected comparison images 25 are arrayed, and/or (ii) a respective second translation, by a respective second integer numbers of pixels, along a second pixel array direction along which the pixels of the selected comparison images 25 are arrayed, the second direction being orthogonal to the first direction. Thus, no rotation of either image in the pair of images being registered occurs. The use of this constraint in the registration process requires the rotational offset threshold $\Theta$ used in process S20 of Figure 4 to be small enough for an image rotation, which would be required to compensate for the rotational offset $\Delta\phi$ between the images in the pair, to result in a displacement at an edge of the image frame which is smaller than a resolution of the ophthalmic imaging device 10. For example, in the case of a FAF imaging device, the resolution is typically limited by the point spread function of the device so the rotational offset threshold $\Theta$ would be set such that rotations required for registration, which produce displacements along an edge of the image frame that are smaller than the width of the point spread function, would be smaller than $\Theta$ (in which case the comparison image in the pair of images would be selected in S30 of Figure 4), while rotations required for registration, which produce displacements along an edge of the image frame that are greater than the width of the point spread function, would be greater than $\Theta$ (in which case the comparison image in the pair of images would not be selected in S30 of Figure 4). By way of an illustrative example, where the images in the sequence are $512 \times 512$ pixels in size and the point spread function for the region 30 is 1.5 pixels wide, as illustrated in Figure 5, then setting $\Theta = 0.3$ degrees would require the maximal displacement caused by the rotation for registration (at an edge of the image frame) to be $\tan(0.3) \cdot 256 = 1.3$ pixels, which is smaller than the width of the point spread function.

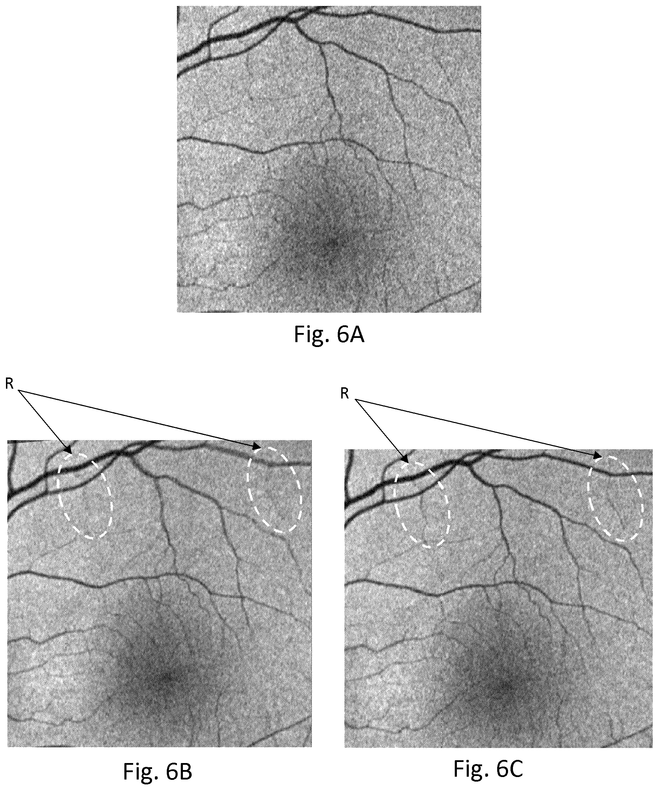

An averaged image, which has been obtained by averaging the example FAF image shown in Figure 6A with another FAF image which is rotationally offset from the image of Figure 6A by 4.5 degrees, is shown in Figure 6B. For comparison, another averaged image, which has been obtained by averaging the FAF image shown in Figure 6A with another FAF image that has a negligible rotational offset from the image of Figure 6A, is shown in Figure 6C. As can be appreciated from a comparison of Figures 6B and 6C, the averaged image of Figure 6C shows anatomical detail in the highlighted regions R that is absent in the averaged image of Figure 6B.

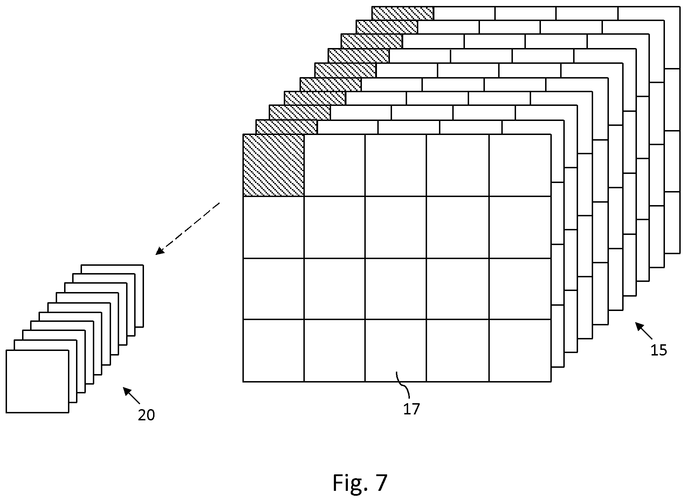

In some cases, a high proportion of the images in the sequence 20 may have intra-frame warping and/or localised regions of micro-warping (where there is micro-misalignment between imaged retinal features) of the kind noted above. In these cases, a relatively low proportion of the images in the sequence of images 20 may be selected in process S30 of Figure 4 for averaging to generate the averaged image 70, and noise suppression in the averaged image 70 may consequently be less effective. In such cases, it may be beneficial to adapt the process of Figure 4 by including, as illustrated schematically in Figure 7, a preliminary process (before S10) of generating each image of the sequence of images 20 by segmenting a respective image of a second sequence of images 15 of a second region of the retina 40, such that the image of the region 30 is a segment 17 of the respective image of the second sequence of images 15. The sequence of images 20 may thus be a sequence of image segments of a second sequence of images 15 acquired by the ophthalmic imaging device 10 imaging a larger region of the retina 40 that includes the region 30, where each image segment 17 has a respective location within the respective image of the second sequence of images 15 which is the same as the respective location of every other image segment within the respective image of the second sequence of images 15. Limiting the subsequent image processing operations in the process of Figure 4 to segments of the second sequence of images 15 may result in a relatively high proportion of images segments being selected in process S30 for averaging to generate the averaged image 70, and consequently less noise in the averaged image 70 than in a case where the images in the second sequence of images 15 are processed as a whole in accordance with the process described above with reference to Figure 4. Since an averaged image 70 based on such a selection of the image segments, although less noisy, will cover only part of the region of the retina 40 that is spanned by the images in the second sequence of images 15, a plurality of other correspondingly located segments 17 of images in the second sequence of images 15 may be processed in the same way to generate additional averaged images that collectively cover more of the second region of the retina 40.

Each image of the second sequence of images 15 may be segmented in the same way into a one- or two-dimensional array of rectangular image tiles (e.g. a two-dimensional array of square image tiles, as illustrated in Figure 7), and each set of correspondingly located image tiles from the segmented images may be processed in accordance with the process described above with reference to Figure 4 to generate a corresponding averaged image tile. The averaged image tiles may then be combined to generate an averaged image of the second region of the retina, which may exhibit varying levels of noise suppression among the component averaged image tiles. The averaged image tiles may also be useful for training a machine learning denoising algorithm to more effectively distinguish between noise and retinal structures, and thus be able to produce de-noised retinal images that retain more of the texture present in the originally acquired images, as described below. It should be noted that the two-dimensional orthogonal array of square image segments 17 in Figure 7 is given by way of an example only, and that the images segments 17 need not be of the same size or shape, and need not be arranged in a regular array.

[Second Example Embodiment]

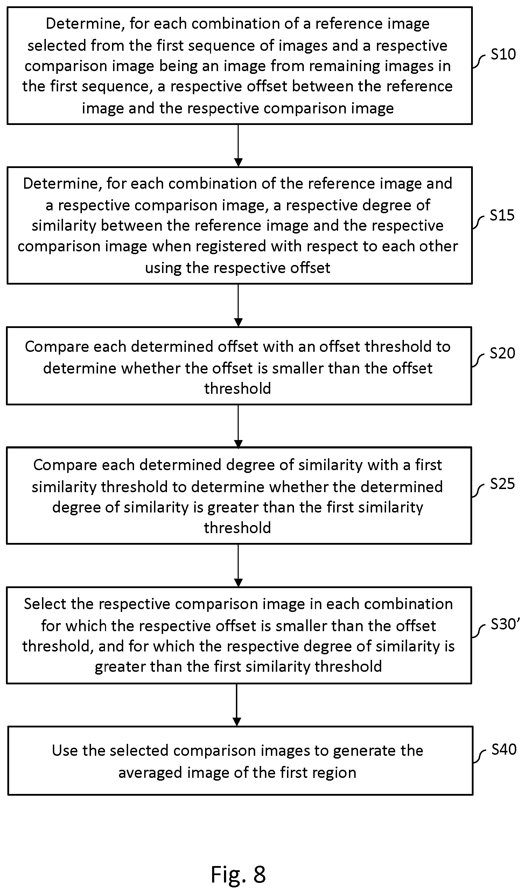

Figure 8 is a flow diagram illustrating a method according to a second example embodiment, by which the data processing apparatus 60 may process the sequence of images 20 to generate an averaged image 70 of the region 30.

Processes S10, S20 and S40 in Figure 8 are the same as those in Figure 4 and will not be described again here. The method of the second example embodiment differs from the method of the first example embodiment by comprising additional processes S15 and S25, as well as a modified form of process S30 (labelled S30' in Figure 8), and these processes will now be described.

In process S15 of Figure 8, the processor 320 determines, for each combination of the reference image $I_{Ref}$ and a respective comparison image $I_{Comp}$, a respective degree of similarity between the reference image $I_{Ref}$ and the respective comparison image $I_{Comp}$ when registered with respect to each other using the respective offset(s), for example the respective translational offset t and the respective rotational offset $\Delta\phi$ of the first example embodiment described above. The respective translational offset t between the reference image $I_{Ref}$ and the respective comparison image $I_{Comp}$ in each combination may, as in the first example embodiment, be determined by calculating a cross-correlation between the reference image $I_{Ref}$ and the respective comparison image $I_{Comp}$. In this case, the respective degree of similarity between the reference image $I_{Ref}$ and the respective comparison image $I_{Comp}$, when registered with respect to each other using the respective translational offset t, may be determined by determining a maximum value of the calculated cross-correlation. It is noted that the order of processes S10 and S15 may be reversed in some example embodiments. Accordingly, once the processor 320 has calculated the cross-correlation between the reference image $I_{Ref}$ and the respective comparison image $I_{Comp}$, the processor 320 may determine the maximum value of the calculated cross-correlation before determining the translational (x-y) offset corresponding to that maximum value. It should be noted that the respective degree of similarity between the reference image $I_{Ref}$ and the respective comparison image $I_{Comp}$, when registered with respect to each other using the respective offset(s), need not be derived from a calculated cross-correlation between the images and may instead be obtained by calculating a sum of squared differences or mutual information based on the images, for example.

In process S25 of Figure 7, the processor 320 compares each degree of similarity determined in process S15 with a first similarity threshold to determine whether the determined degree of similarity is greater than the first similarity threshold. It is noted that the order of processes S20 and S25 may be reversed in some example embodiments.

In process S30' of Figure 8, the processor 320 selects the respective comparison image $I_{Comp}$ in each combination for which the reference image $I_{Ref}$ and the respective comparison image $I_{Comp}$ have been determined to have a respective offset therebetween which is smaller than the offset threshold (i.e. each combination which satisfies the condition in S30 of Figure 4), and a respective degree of similarity, when registered with respect to each other using the respective offset, which is greater than the first similarity threshold. The latter additional selection criterion helps avoid comparison images $I_{Comp}$ that have intra-frame non-affine warping and/or comparison images $I_{Comp}$ having micro-warping (i.e. where misalignment of retinal features is limited to one or more localised regions) being selected for averaging to generate the averaged image 70, as such images tend to be relatively poorly correlated to the reference image $I_{Ref}$ even when the offset(s) between them and the reference image $I_{Ref}$ are determined to be smaller than the offset threshold(s). Additional processes S15 and S25, and modified process S30' of Figure 8, may thus assist in avoiding loss of valuable clinical data such as texture in the averaged image 70. The first similarity threshold may be set by trial and error, observing the effect of adjustments of the first similarity threshold on the amount of texture present in the averaged image 70, for example.

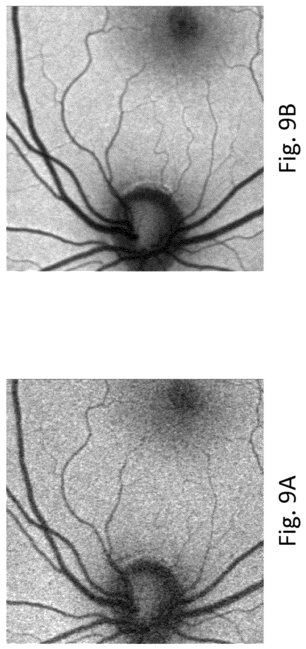

Figure 9A shows an example FAF image acquired by the ophthalmic imaging device 10. For comparison, Figure 9B shows an example of the averaged image 70, which has been obtained by averaging 10 FAF images, including the FAF image of Figure 9A, that have been selected from a sequence of FAF images in accordance with the method described herein with reference to Figure 8. Figure 9B shows the averaged image to have less noise than the FAF image of Figure 9A whilst retaining much of the texture of the FAF image of Figure 9A (most apparent in the light grey regions of the image).

Referring again to Figure 8, in process S25, the processor 320 may also compare each determined degree of similarity with a second similarity threshold to determine whether the determined degree of similarity is smaller than the second similarity threshold, the second similarity threshold being greater than the first similarity threshold. In this case, the processor 320 may perform a modified version of process S30', by selecting the respective comparison image $I_{Comp}$ in each combination for which the reference image $I_{Ref}$ and the respective comparison image $I_{Comp}$ have been determined to have a respective offset therebetween which is smaller than the offset threshold, and a respective degree of similarity, when the reference image $I_{Ref}$ and the respective comparison image $I_{Comp}$ are registered with respect to each other using the respective offset, which is greater than the first similarity threshold and smaller than the second similarity threshold. Comparison images may thus be selected for averaging to produce the averaged image 70 using the same criteria as in the first example embodiment, and the additional criterion that the degree of similarity is within a predefined range of values, i.e. between the first similarity threshold and the second similarity threshold. The exclusion from the selection of comparison images that yield excessively high determined degrees of similarity (as determined by comparison with an appropriate value for the second similarity threshold set by trial and error), as well as those that yield insufficiently high determined degrees of similarity (as determined by comparison with an appropriate value for the first similarity threshold set by trial and error), was found by the inventor to improve the retention of texture in the averaged image 70. Without wishing to be bound by theory, the reason for this may be understood as follows. There is a certain amount of information in any imaged region on the retina, and each single image can be considered to capture only a proportion of that information space, due to factors such as subtle variations in lighting, changes in the scanning system (such as line start positioning and subtle changes in related optical paths) and also other complex effects as to the way light scattering works from layers in the retina. Statistically, images of the region that differ slightly in information content will tend to produce an averaged image that overall reassembles a greater proportion of the total amount of information available than either of the individual images. From another perspective, very highly correlated images with exactly the same information content would not produce an averaged image with more of the information than either individual image but will still contain noise

[Third Example Embodiment]

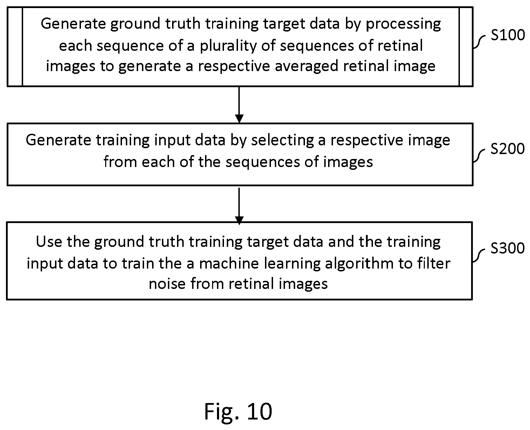

Figure 10 is a flow diagram illustrating a method according to a third example embodiment, by which the data processing apparatus 60 trains a machine learning (ML) algorithm to filter noise from retinal images. The ML algorithm may be any kind of supervised ML algorithm known to those versed in the art which is suitable for removing noise of one or more different types from FAF images or any other kind of retinal images, once it has been trained to perform this task using labelled training data. The ML algorithm may comprise a convolutional neural network (CNN), as in the present example embodiment. The article titled "Image De-Noising With Machine Learning: A Review" by R. S. Thakur et al., IEEE Access, Vol. 9, pages 93338-93363 (2021), the contents of which are incorporated herein by reference in their entirety, describes various state-of-the-art machine-learning-based image de-noisers like dictionary learning models, convolutional neural networks and generative adversarial networks for a range of noises like Gaussian, Impulse, Poisson, Mixed and Real-World noises. The ML algorithm may be of any of the different kinds described in this article or otherwise known to those versed in the art.

In process S100 of Figure 10, the processor 320 of the data processing apparatus 60 generates ground truth training target data by processing each sequence 20 of a plurality of sequences of retinal images as described in any of the foregoing example embodiments or their variants to generate a respective averaged retinal image.

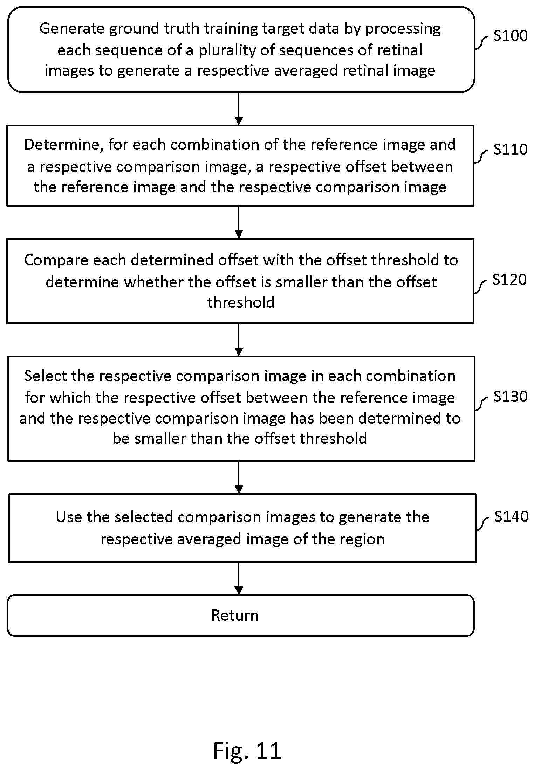

Figure 11 is a flow diagram summarising the method by which the processor 320 generates each of the averaged retinal images from the respective sequence of retinal images in the present example embodiment.

In process S110 of Figure 11, the processor 320 determines, for each combination of the reference image $I_{Ref}$ and a respective comparison image $I_{Comp}$ being an image from remaining images in the sequence 20, a respective offset between the reference image $I_{Ref}$ and the respective comparison image $I_{Comp}$.

In process S120 of Figure 11, the processor 320 compares each determined offset with the offset threshold to determine whether the offset is smaller than the offset threshold.

In process S130 of Figure 11, the processor 320 selects the respective comparison image $I_{Comp}$ in each combination for which the respective offset between the reference image $I_{Ref}$ and the respective comparison image $I_{Comp}$ has been determined to be smaller than the offset threshold.

In process S140 of Figure 11, the processor 320 uses the selected comparison images 25 to generate the respective averaged image 70 of the region 30.

As explained above, the offset threshold is such that, where the sequence 20 comprises at least one image which is offset from the reference image $I_{Ref}$ by an offset greater than the threshold, and images that are offset from the reference image $I_{Ref}$ by respective offsets that are smaller than the threshold, the averaged image 70 shows more texture which is indicative of a structure in the first region 30 of the retina 40 than a reference averaged image generated from the images in the sequence of images 20.

Referring again to Figure 10, in process S200, the processor 320 generates training input data by selecting a respective image from each of the sequences of images.

Then, in process S300 of Figure 10, the processor 320 uses the ground truth training target data and the training input data to train the ML algorithm to filter noise from retinal images using techniques well known to those versed in the art.

Some of the example embodiments described above are summarised in following numbered clauses E1 to E14.

E1. A data processing apparatus 60 arranged to process a first sequence of images 20 of a region 30 of a retina 40 of an eye 50 to generate an averaged image 70 of the region 30, the data processing apparatus 60 comprising at least one processor 320 and at least one memory 340 storing computer-readable instructions that, when executed by the at least one processor 320, cause the at least one processor 320 to:

determine, for each combination of a reference image $I_{Ref}$ selected from the first sequence of images 20 and a respective comparison image $I_{Comp}$ being an image from remaining images in the first sequence of images 20, a respective offset between the reference image $I_{Ref}$ and the respective comparison image $I_{Comp}$;

compare each determined offset with an offset threshold to determine whether the offset is smaller than the offset threshold;

select the respective comparison image $I_{Comp}$ in each combination for which the respective offset between the reference image $I_{Ref}$ and the respective comparison image $I_{Comp}$ has been determined to be smaller than the offset threshold; and

use the selected comparison images 25 to generate the averaged image 70 of the region 30,

wherein the offset threshold is such that, where the first sequence of images 20 comprises at least one image which is offset from the reference image $I_{Ref}$ by an offset greater than the threshold, and images that are offset from the reference image $I_{Ref}$ by respective offsets that are smaller than the threshold, the averaged image 70 shows more texture which is indicative of a structure in the first region 30 of the retina 40 than a reference averaged image generated from the images in the first sequence of images 20.

E2. The data processing apparatus 60 of E1, wherein

the respective offset determined for each combination of the reference image $I_{Ref}$ and the respective comparison image $I_{Comp}$ comprises a translational offset, and

the computer-readable instructions, when executed by the at least one processor 320, cause the at least one processor 320 to:

compare each determined offset with the offset threshold to determine whether the offset is smaller than the offset threshold by comparing each translational offset with a translational offset threshold to determine whether the translational offset is smaller than the translational offset threshold; and

select the respective comparison image $I_{Comp}$ in each combination for which the respective offset between the reference image $I_{Ref}$ and the respective comparison image $I_{Comp}$ has been determined to be smaller than the offset threshold by selecting the respective comparison image $I_{Comp}$ in each combination for which the respective translational offset has been determined to be smaller than the translational offset threshold.

E3. The data processing apparatus 60 of E2, wherein the computer-readable instructions, when executed by the at least one processor 320, cause the at least one processor 320 to determine the respective translational offset between the reference image $I_{Ref}$ and the respective comparison image $I_{Comp}$ in each combination by one of:

calculating a cross-correlation using the reference image $I_{Ref}$ and the respective comparison image $I_{Comp}$; and

calculating an inverse Fourier transform of a normalized cross-power spectrum calculated using the reference image $I_{Ref}$ and the respective comparison image $I_{Comp}$.

E4. The data processing apparatus 60 of any of E1 to E3, wherein

the respective offset determined for each combination of the reference image $I_{Ref}$ and the respective comparison image $I_{Comp}$ comprises a rotational offset, and

the computer-readable instructions, when executed by the at least one processor 320, cause the at least one processor 320 to:

compare each determined offset with the offset threshold to determine whether the offset is smaller than the offset threshold by comparing each rotational offset with a rotational offset threshold to determine whether the rotational offset is smaller than the rotational offset threshold; and
select the respective comparison image $I_{Comp}$ in each combination for which the respective offset between the reference image $I_{Ref}$ and the respective comparison image $I_{Comp}$ has been determined to be smaller than the offset threshold by selecting the respective comparison image $I_{Comp}$ in each combination for which the respective rotational offset has been determined to be smaller than the rotational offset threshold.

E5. The data processing apparatus 60 of E4, wherein the computer-readable instructions, when executed by the at least one processor 320, cause the at least one processor 320 to determine the respective rotational offset between the reference image $I_{Ref}$ and the respective comparison image $I_{Comp}$ in each combination by one of:

calculating cross-correlations using rotated versions of the respective comparison image $I_{Comp}$; and

calculating an inverse Fourier transform of a normalized cross-power spectrum calculated using polar transformations of the reference image $I_{Ref}$ and the respective comparison.

E6. The data processing apparatus 60 of any of E1 to E5, wherein the computer-readable instructions, when executed by the at least one processor 320, cause the at least one processor 320 to use the selected comparison images 25 to generate the averaged image of the first region by:

registering the selected comparison images 25 with respect to one another, wherein registering each pair of the selected comparison images 25 comprises redistributing pixel values of one of the images in the pair according to a respective geometric transformation between image coordinate systems of the images in the pair; and

generating the averaged image 70 of the first region by averaging the registered images.

E7. The data processing apparatus 60 of E6, wherein the respective geometric transformation between the image coordinate systems of the images in each pair of the selected comparison images 25 consists of at least one of:

a respective first translation, by a respective first integer numbers of pixels, along a first pixel array direction along which pixels of the selected comparison images 25 are arrayed; and

a respective second translation, by a respective second integer numbers of pixels, along a second pixel array direction along which the pixels of the selected comparison images 25 are arrayed.

E8. The data processing apparatus 60 of any of E1 to E7, wherein the computer-readable instructions, when executed by the at least one processor 320, further cause the at least one processor 320 to:

determine, for each combination of the reference image $I_{Ref}$ and a respective comparison image $I_{Comp}$, a respective degree of similarity between the reference image $I_{Ref}$ and the respective comparison image $I_{Comp}$ when registered with respect to each other using the respective offset; and

compare each determined degree of similarity with a first similarity threshold to determine whether the determined degree of similarity is greater than the first similarity threshold,

wherein the computer-readable instructions, when executed by the at least one processor 320, cause the at least one processor 320 to select the respective comparison image $I_{Comp}$ in each combination for which the reference image $I_{Ref}$ and the respective comparison image $I_{Comp}$ have been determined to have a respective offset therebetween which is smaller than the offset threshold, and a respective degree of similarity, when registered with respect to each other using the respective offset, which is greater than the first similarity threshold.

E9. The data processing apparatus 60 of E8, wherein

the computer-readable instructions, when executed by the at least one processor 320, further cause the at least one processor 320 to compare each determined degree of similarity with a second similarity threshold to determine whether the determined degree of similarity is smaller than the second similarity threshold, the second similarity threshold being greater than the first similarity threshold, and

the computer-readable instructions, when executed by the at least one processor 320, cause the at least one processor 320 to select the respective comparison image $I_{Comp}$ in each combination for which the reference image $I_{Ref}$ and the respective comparison image $I_{Comp}$ have been determined to have a respective offset therebetween which is smaller than the offset threshold, and a respective degree of similarity, when registered with respect to each other using the respective offset, which is greater than the first similarity threshold and smaller than the second similarity threshold.

E10. The data processing apparatus 60 of E8 when dependent on E2, or on E9 when dependent on E2, wherein the computer-readable instructions, when executed by the at least one processor 320, cause the at least one processor 320 to:

determine the respective translational offset between the reference image $I_{Ref}$ and the respective comparison image $I_{Comp}$ in each combination by calculating a cross-correlation using the reference image $I_{Ref}$ and the respective comparison image $I_{Comp}$, and

determine the respective degree of similarity between the reference image $I_{Ref}$ and the respective comparison image $I_{Comp}$ when registered with respect to each other using the respective translational offset, by determining a maximum value of the calculated cross-correlation.

E11. The data processing apparatus 60 of any of E1 to E10, wherein the computer-readable instructions, when executed by the at least one processor 320, further cause the at least one processor 320 to generate each image of the first sequence of images 20 by segmenting a respective image of a second sequence of images of a second region of the retina 40, such that the image of the first region 30 is a segment of the respective image of the second sequence of images.

E12. The data processing apparatus 60 of any of E1 to E11, wherein the first sequence of images 20 comprises a sequence of autofluorescence images of the first region 30 of the retina 40 of the eye 50.

E13. The data processing apparatus 60 of any of E1 to E12, wherein the computer-readable instructions, when executed by the at least one processor 320, further cause the at least one processor 320 to train a machine learning algorithm to filter noise from retinal images, by:

generating ground truth training target data by processing each sequence 20 of a plurality of sequences of retinal images to generate respective averaged retinal images, each of the averaged retinal images being generated by:

determining, for each combination of the reference image $I_{Ref}$ and a respective comparison image $I_{Comp}$ being an image from remaining images in the sequence 20, a respective offset between the reference image $I_{Ref}$ and the respective comparison image $I_{Comp}$;

comparing each determined offset with the offset threshold to determine whether the offset is smaller than the offset threshold;

selecting the respective comparison image $I_{Comp}$ in each combination for which the respective offset between the reference image $I_{Ref}$ and the respective comparison image $I_{Comp}$ has been determined to be smaller than the offset threshold; and

using the selected comparison images 25 to generate the respective averaged image 70 of the region 30,

wherein the offset threshold is such that, where the sequence 20 comprises at least one image which is offset from the reference image $I_{Ref}$ by an offset greater than the threshold, and images that are offset from the reference image $I_{Ref}$ by respective offsets that are smaller than the threshold, the averaged image 70 shows more texture which is indicative of a structure in the first region 30 of the retina 40 than a reference averaged image generated from the images in the sequence of images 20;

generating training input data by selecting a respective image from each of the sequences of images; and

using the ground truth training target data and the training input data to train the machine learning algorithm to filter noise from retinal images.

E14. An ophthalmic imaging system 100 comprising:

an ophthalmic imaging device 10 arranged to acquire a sequence of images 20 of a region 30 of a retina 40 of an eye 50; and

a data processing apparatus 60 according to any of E1 to E13, which is arranged to process the sequence of images 20 acquired by the ophthalmic imaging device 10 to generate an averaged image 70 of the region 30 of the retina 40.

In the foregoing description, example aspects are described with reference to several example embodiments. Accordingly, the specification should be regarded as illustrative, rather than restrictive. Similarly, the figures illustrated in the drawings, which highlight the functionality and advantages of the example embodiments, are presented for example purposes only. The architecture of the example embodiments is sufficiently flexible and configurable, such that it may be utilized in ways other than those shown in the accompanying figures.

Some aspects of the examples presented herein may be provided as a computer program, or software, such as one or more programs having instructions or sequences of instructions, included or stored in an article of manufacture such as a machine-accessible or machine-readable medium, an instruction store, or computer-readable storage device, each of which can be non-transitory, in one example embodiment. The program or instructions on the non-transitory machine-accessible medium, machine-readable medium, instruction store, or computer-readable storage device, may be used to program a computer system or other electronic device. The machine- or computer-readable medium, instruction store, and storage device may include, but are not limited to, floppy diskettes, optical disks, and magneto-optical disks or other types of media/machine-readable medium/instruction store/storage device suitable for storing or transmitting electronic instructions. The techniques described herein are not limited to any particular software configuration. They may find applicability in any computing or processing environment. The terms "computer-readable", "machine-accessible medium", "machine-readable medium", "instruction store", and "computer-readable storage device" used herein shall include any medium that is capable of storing, encoding, or transmitting instructions or a sequence of instructions for execution by the machine, computer, or computer processor and that causes the machine/computer/computer processor to perform any one of the methods described herein. Furthermore, it is common in the art to speak of software, in one form or another (e.g., program, procedure, process, application, module, unit, logic, and so on), as taking an action or causing a result. Such expressions are merely a shorthand way of stating that the execution of the software by a processing system causes the processor to perform an action to produce a result.

Some or all of the functionality of the data processing apparatus 60 may also be implemented by the preparation of application-specific integrated circuits, field-programmable gate arrays, or by interconnecting an appropriate network of conventional component circuits.

A computer program product may be provided in the form of a storage medium or media, instruction store(s), or storage device(s), having instructions stored thereon or therein which can be used to control, or cause, a computer or computer processor to perform any of the procedures of the example embodiments described herein. The storage medium/instruction store/storage device may include, by example and without limitation, an optical disc, a ROM, a RAM, an EPROM, an EEPROM, a DRAM, a VRAM, a flash memory, a flash card, a magnetic card, an optical card, nanosystems, a molecular memory integrated circuit, a RAID, remote data storage/archive/warehousing, and/or any other type of device suitable for storing instructions and/or data.

Stored on any one of the computer-readable medium or media, instruction store(s), or storage device(s), some implementations include software for controlling both the hardware of the system and for enabling the system or microprocessor to interact with a human user or other mechanism utilizing the results of the example embodiments described herein. Such software may include without limitation device drivers, operating systems, and user applications. Ultimately, such computer-readable media or storage device(s) further include software for performing example aspects of the invention, as described above.

Included in the programming and/or software of the system are software modules for implementing the procedures described herein. In some example embodiments herein, a module includes software, although in other example embodiments herein, a module includes hardware, or a combination of hardware and software.

While various example embodiments of the present invention have been described above, it should be understood that they have been presented by way of example, and not limitation. It will be apparent to persons skilled in the relevant art(s) that various changes in form and detail can be made therein. Thus, the present invention should not be limited by any of the above-described example embodiments, but should be defined only in accordance with the following claims.

While this specification contains many specific embodiment details, these should not be construed as limitations on the scope of any inventions or of what may be claimed, but rather as descriptions of features specific to particular embodiments described herein. The invention is defined by the appended claims.

In certain circumstances, multitasking and parallel processing may be advantageous. Moreover, the separation of various components in the embodiments described above should not be understood as requiring such separation in all embodiments, and it should be understood that the described program components and systems can generally be integrated together in a single software product or packaged into multiple software products.

**Claims**

1. A computer-implemented method of processing a first sequence of images (20) of a first region (30) of the retina (40) of an eye (50) to generate an averaged image (70) of the first region (30), the method comprising:

    determining (S10), for each combination of a reference image ($I_{Ref}$) selected from the first sequence of images (20) and a respective comparison image ($I_{Comp}$) being an image from remaining images in the first sequence (20), a respective translational offset (t) between the reference image ($I_{Ref}$) and the respective comparison image ($I_{Comp}$);
    comparing (S20) the magnitude of each determined translational offset (t) with a translational offset threshold (T) to determine whether the translational offset (t) is smaller than the translational offset threshold (T);
    selecting (S30) the respective comparison image ($I_{Comp}$) in each combination for which the respective translational offset (t) between the reference image ($I_{Ref}$) and the respective comparison image ($I_{Comp}$) has been determined to be smaller than the translational offset threshold (T); and
    using (S40) the selected comparison images (25) to generate the averaged image (70) of the first region (30), by:

        registering the selected comparison images (25) with respect to one another; and
        generating the averaged image (70) of the first region (30) by averaging the registered images, such that the respective pixel value of each pixel in the averaged image (70) is a mean of the pixel values of correspondingly located pixels in the registered images that correspond to respective common location in the region (30) of the retina (40),

    wherein the translational offset threshold (T) is such that, where the first sequence of images (20) comprises at least one image which is offset from the reference image ($I_{Ref}$) by a translational offset greater than the translational offset threshold (T), and images that are offset from the reference image ($I_{Ref}$) by respective translational offsets (t) that are smaller than the translational offset threshold (T), the averaged image (70) shows more texture which is indicative of a structure in the first region (30) of the retina (40) than a reference averaged image (75) generated from the images in the first sequence of images (20).

2. The computer-implemented method of Claim 1, wherein the respective translational offset (t) between the reference image ($I_{Ref}$) and the respective comparison image ($I_{Comp}$) in each combination is determined (S10) by one of:

    calculating a cross-correlation using the reference image ($I_{Ref}$) and the respective comparison image ($I_{Comp}$); or
    calculating an inverse Fourier transform of a normalized cross-power spectrum calculated using the reference image ($I_{Ref}$) and the respective comparison image ($I_{Comp}$).

3. The computer-implemented method of Claim 1 or Claim 2, further comprising:

    determining, for each combination of the reference image ($I_{Ref}$) and the respective comparison image ($I_{Comp}$), a respective rotational offset ($\Delta\phi$) between the reference image ($I_{Ref}$) and the respective comparison image ($I_{Comp}$); and comparing the magnitude of each rotational offset ($\Delta\phi$) with a rotational offset threshold ($\Theta$) to determine whether the rotational offset ($\Delta\phi$) is smaller than the rotational offset threshold ($\Theta$),
    wherein the selecting (S30) comprises selecting the respective comparison image ($I_{Comp}$) in each combination for which the respective rotational offset ($\Delta\phi$) has been determined to be smaller than the rotational offset threshold

($\Theta$).

4. The computer-implemented method of Claim 3, wherein the respective rotational offset ($\Delta\phi$) between the reference image ($I_{Ref}$) and the respective comparison image ($I_{Comp}$) in each combination is determined by one of:

    calculating cross-correlations using rotated versions of the respective comparison image ($I_{Comp}$); or
    calculating an inverse Fourier transform of a normalized cross-power spectrum calculated using transformations of the reference image ($I_{Ref}$) and the respective comparison image ($I_{Comp}$) into polar coordinates.

5. The computer-implemented method of any preceding claim, wherein the selected comparison images (25) are registered with respect to one another by redistributing pixel values of one of the images in the pair according to a respective geometric transformation between image coordinate systems of the images in the pair.

6. The computer-implemented method of Claim 5, wherein the respective geometric transformation between the image coordinate systems of the images in each pair of the selected comparison images (25) consists of at least one of:

    a respective translation, by a respective first integer number of pixels, along a first pixel array direction along which pixels in the selected comparison images (25) are arrayed; or
    a respective translation, by a respective second integer number of pixels, along a second pixel array direction along which the pixels in the selected comparison images (25) are arrayed, the second pixel array direction being orthogonal to the first pixel array direction.

7. The computer-implemented method of any preceding claim, further comprising:

    determining (S15), for each combination of the reference image ($I_{Ref}$) and a respective comparison image ($I_{Comp}$), a respective degree of similarity between the reference image ($I_{Ref}$) and the respective comparison image ($I_{Comp}$) when registered with respect to each other using the respective translational offset; and
    comparing (S25) each determined degree of similarity with a first similarity threshold to determine whether the determined degree of similarity is greater than the first similarity threshold,
    wherein the selecting comprises selecting (S30') the respective comparison image ($I_{Comp}$) in each combination for which the reference image ($I_{Ref}$) and the respective comparison image ($I_{Comp}$) have been determined to have a respective degree of similarity, when registered with respect to each other using the respective offset, which is greater than the first similarity threshold.

8. The computer-implemented method of Claim 7, further comprising comparing each determined degree of similarity with a second similarity threshold to determine whether the determined degree of similarity is smaller than the second similarity threshold, the second similarity threshold being greater than the first similarity threshold, wherein the selecting comprises selecting the respective comparison image ($I_{Comp}$) in each combination for which the reference image ($I_{Ref}$) and the respective comparison image ($I_{Comp}$) have been determined to have a respective degree of similarity, when registered with respect to each other using the respective offset, which is greater than the first similarity threshold and smaller than the second similarity threshold.

9. The computer-implemented method of Claim 7 or Claim 8, wherein

    the respective translational offset (t) between the reference image ($I_{Ref}$) and the respective comparison image ($I_{Comp}$) in each combination is determined by calculating a cross-correlation using the reference image ($I_{Ref}$) and the respective comparison image ($I_{Comp}$), and
    the respective degree of similarity between the reference image ($I_{Ref}$) and the respective comparison image ($I_{Comp}$), when registered with respect to each other using the respective translational offset (t), is determined by determining a maximum value of the calculated cross-correlation.

10. The computer-implemented method of any preceding claim, further comprising generating each image of the first sequence of images (20) by segmenting a respective image of a second sequence of images of a second region of the retina (40), such that the image of the first region (30) is a segment of the respective image of the second sequence of images.

11. The computer-implemented method of any preceding claim, wherein the first sequence of images (20) comprises a sequence of autofluorescence images of the first region (30) of the retina (40) of the eye (50).

12. A computer-implemented method of training a machine learning algorithm to filter noise from retinal images, the method comprising:

generating (S100) ground truth training target data by processing each sequence of a plurality of sequences of retinal images to generate a respective averaged retinal image, wherein each averaged retinal image is generated in accordance with the computer-implemented method of any preceding claim;

generating (S200) training input data by selecting a respective image from each of the sequences of images; and

using (S300) the ground truth training target data and the training input data to train the machine learning algorithm to filter noise from retinal images.

13. A computer program (345) comprising computer-readable instructions that, when executed by at least one processor (320), cause the at least one processor (320) to execute a method according to any preceding claim.

14. A data processing apparatus (60) arranged to process a sequence of images (20) of a region (30) of the retina (40) of an eye (50) to generate an averaged image (70) of the region (30), the data processing apparatus (60) comprising at least one processor (320) and at least one memory (340) storing computer-readable instructions that, when executed by the at least one processor (320), cause the at least one processor (320) to:

determine, for each combination of a reference image ($I_{Ref}$) selected from the sequence of images and a respective comparison image ($I_{Comp}$) being an image from remaining images in the sequence (20), a respective translational offset (t) between the reference image ($I_{Ref}$) and the respective comparison image ($I_{Comp}$);

compare the magnitude of each determined translational offset (t) with a translational offset threshold (T) to determine whether the translational offset (t) is smaller than the translational offset threshold (T);

select the respective comparison image ($I_{Comp}$) in each combination for which the respective translational offset (t) between the reference image ($I_{Ref}$) and the respective comparison image ($I_{Comp}$) has been determined to be smaller than the translational offset threshold (T); and

use the selected comparison images (25) to generate the averaged image (70) of the region (30), by:

registering the selected comparison images (25) with respect to one another; and

generating the averaged image (70) of the first region (30) by averaging the registered images, such that the respective pixel value of each pixel in the averaged image (70) is a mean of the pixel values of correspondingly located pixels in the registered images that correspond to respective common location in the region (30) of the retina (40),

wherein the translational offset threshold (T) is such that, where the sequence of images (20) comprises at least one image which is offset from the reference image ($I_{ref}$) by a translational offset greater than the translational offset threshold (T), and images that are offset from the reference image ($I_{Ref}$) by respective translational offsets that are smaller than the translational offset threshold (T), the averaged image (70) shows more texture which is indicative of a structure in the first region (30) of the retina (40) than a reference averaged image (75) generated from the images in the sequence of images (20).

15. An ophthalmic imaging system (100) comprising:

an ophthalmic imaging device (10) arranged to acquire a sequence of images (20) of a region (30) of the retina (40) of an eye (50); and

a data processing apparatus (60) according to Claim 14, which is arranged to process the sequence of images (20) acquired by the ophthalmic imaging device (10) to generate an averaged image (70) of the region (30) of the retina (40).

**Patentansprüche**

1. Ein computerimplementiertes Verfahren zum Verarbeiten einer ersten Folge von Bildern (20) eines ersten Bereichs (30) der Netzhaut (40) eines Auges (50), um ein gemitteltes Bild (70) des ersten Bereichs (30) zu erzeugen, wobei das Verfahren umfasst

Bestimmen (S10), für jede Kombination eines Referenzbildes ($I_{Ref}$), das aus der ersten Sequenz von Bildern (20) ausgewählt ist, und eines jeweiligen Vergleichsbildes ($I_{Comp}$), das ein Bild aus verbleibenden Bildern in der ersten Sequenz (20) ist, eines jeweiligen Translationsversatzes (t) zwischen dem Referenzbild ($I_{Ref}$) und dem jeweiligen Vergleichsbild ($I_{Comp}$);

Vergleichen (S20) der Größe jedes bestimmten Translationsversatz (t) mit einem Translationsversatz-Schwellenwert (T), um zu bestimmen, ob der Translationsversatz (t) kleiner als der Translationsversatz-Schwellenwert (T) ist;

Auswählen (S30) des jeweiligen Vergleichsbildes ($I_{Comp}$) in jeder Kombination, für die der jeweilige Translationsversatz (t) zwischen dem Referenzbild ($I_{Ref}$) und dem jeweiligen Vergleichsbild ($I_{Comp}$) als kleiner als der Translationsversatz-Schwellenwert (T) bestimmt worden ist; und

Verwenden (S40) der ausgewählten Vergleichsbilder (25), um das gemittelte Bild (70) des ersten Bereichs (30) zu erzeugen, durch:

Ausrichten der ausgewählten Vergleichsbilder (25) in Bezug aufeinander; und

Erzeugen des gemittelten Bildes (70) des ersten Bereichs (30) durch Mitteln der registrierten Bilder, derart, dass der jeweilige Pixelwert jedes Pixels in dem gemittelten Bild (70) ein Mittelwert der Pixelwerte von entsprechend angeordneten Pixeln in den registrierten Bildern ist, die einem jeweiligen gemeinsamen Ort in dem Bereich (30) der Retina (40) zugehören,

wobei der Translationsversatz-Schwellenwert (T) derart ist, dass, wenn die erste Folge von Bildern (20) mindestens ein Bild umfasst, das gegenüber dem Referenzbild ($I_{Ref}$) um einen Translationsversatz versetzt ist, der größer ist als der Translationsversatz-Schwellenwert (T), und Bilder, die gegenüber dem Referenzbild ($I_{Ref}$) um entsprechende Translationsoffsets (t) versetzt sind, die kleiner als der Translationsoffset-Schwellenwert (T) sind, das gemittelte Bild (70) mehr Textur zeigt, die eine Struktur in der ersten Region (30) der Retina (40) angibt, als ein gemitteltes Referenzbild (75), das aus den Bildern der ersten Bildsequenz (20) erzeugt wird.

2. Computerimplementiertes Verfahren nach Anspruch **1**, wobei der jeweilige Translationsversatz (t) zwischen dem Referenzbild ($I_{Ref}$) und dem jeweiligen Vergleichsbild ($I_{Comp}$) in jeder Kombination bestimmt wird (S10) durch eines von Berechnen einer Kreuzkorrelation unter Verwendung des Referenzbildes ($I_{Ref}$) und des jeweiligen Vergleichsbildes ($IC_{omp}$); oder

Berechnung einer inversen Fourier-Transformation eines normalisierten Kreuzleistungsspektrums, das unter Verwendung des Referenzbildes ($I_{Ref}$) und des jeweiligen Vergleichsbildes ($I_{Comp}$) berechnet wird.

3. Das computerimplementierte Verfahren nach Anspruch 1 oder Anspruch 2, das ferner Folgendes umfasst:

Bestimmen eines jeweiligen Rotations-Offsets ($\Delta\varphi$) zwischen dem Referenzbild ($I_{Ref}$) und dem jeweiligen Vergleichsbild ($I_{Comp}$) für jede Kombination aus dem Referenzbild ($I_{Ref}$) und dem jeweiligen Vergleichsbild ($I_{Comp}$); und

Vergleichen der Größe jedes Rotationsversatzes ($\Delta\varphi$) mit einem Rotationsversatz-Schwellenwert ($\Theta$), um zu bestimmen, ob der Rotationsversatz ($\Delta\varphi$) kleiner als der Rotationsversatz-Schwellenwert ($\Theta$) ist,

wobei das Auswählen (S30) das Auswählen des jeweiligen Vergleichsbildes ($I_{Comp}$) in jeder Kombination umfasst, für die der jeweilige Drehversatz ($\Delta\varphi$) als kleiner als der Drehversatz-Schwellenwert ($\Theta$) bestimmt worden ist.

4. Computerimplementiertes Verfahren nach Anspruch 3, wobei der jeweilige Drehversatz ($\Delta\varphi$) zwischen dem Referenzbild ($I_{Ref}$) und dem jeweiligen Vergleichsbild ($I_{Comp}$) in jeder Kombination bestimmt wird durch eine der folgenden Maßnahmen

Berechnung von Kreuzkorrelationen unter Verwendung gedrehter Versionen des jeweiligen Vergleichsbildes ($I_{Comp}$); oder

Berechnung einer inversen Fourier-Transformation eines normalisierten Kreuzleistungsspektrums, das unter Verwendung von Transformationen des Referenzbildes ($I_{Ref}$) und des jeweiligen Vergleichsbildes ($I_{Comp}$) in Polarkoordinaten berechnet wird.

5. Computerimplementiertes Verfahren nach einem der vorhergehenden Ansprüche, bei dem die ausgewählten Vergleichsbilder (25) durch Umverteilung der Pixelwerte eines der Bilder des Paares gemäß einer entsprechenden geometrischen Transformation zwischen den Bildkoordinatensystemen der Bilder des Paares miteinander registriert werden.

6. Computerimplementiertes Verfahren nach Anspruch 5, wobei die jeweilige geometrische Transformation zwischen den Bildkoordinatensystemen der Bilder in jedem Paar der ausgewählten Vergleichsbilder (25) aus mindestens einem der folgenden Punkte besteht

einer jeweiligen Translation um eine jeweilige erste ganzzahlige Anzahl von Pixeln entlang einer ersten Pixelanordnungsrichtung, entlang derer Pixel in den ausgewählten Vergleichsbildern (25) angeordnet sind; oder einer jeweiligen Verschiebung um eine jeweilige zweite ganzzahlige Anzahl von Pixeln entlang einer zweiten Pixelarray-Richtung, entlang der die Pixel in den ausgewählten Vergleichsbildern (25) angeordnet sind, wobei die zweite Pixelarray-Richtung orthogonal zu der ersten Pixelarray-Richtung ist.

7. Das computerimplementierte Verfahren nach einem der vorhergehenden Ansprüche, das ferner Folgendes umfasst:

Bestimmen (S15) eines jeweiligen Ähnlichkeitsgrades zwischen dem Referenzbild ($I_{Ref}$) und dem jeweiligen Vergleichsbild ($I_{Comp}$) für jede Kombination des Referenzbildes ($I_{Ref}$) und eines jeweiligen Vergleichsbildes ($I_{Comp}$), wenn diese unter Verwendung des jeweiligen Translations-Offsets in Bezug zueinander registriert sind; und
Vergleichen (S25) jedes bestimmten Ähnlichkeitsgrades mit einem ersten Ähnlichkeitsschwellenwert, um zu bestimmen, ob der bestimmte Ähnlichkeitsgrad größer als der erste Ähnlichkeitsschwellenwert ist, wobei das Auswählen das Auswählen (S30') des jeweiligen Vergleichsbildes ($I_{Comp}$) in jeder Kombination umfasst, für die festgestellt wurde, dass das Referenzbild ($I_{Ref}$) und das jeweilige Vergleichsbild ($I_{Comp}$) einen jeweiligen Ähnlichkeitsgrad aufweisen, wenn sie unter Verwendung des jeweiligen Versatzes in Bezug zueinander registriert werden, der größer als der erste Ähnlichkeitsschwellenwert ist.

8. Computerimplementiertes Verfahren nach Anspruch 7, ferner umfassend das Vergleichen jedes ermittelten Ähnlichkeitsgrades mit einem zweiten Ähnlichkeitsschwellenwert, um zu bestimmen, ob der ermittelte Ähnlichkeitsgrad kleiner als der zweite Ähnlichkeitsschwellenwert ist, wobei der zweite Ähnlichkeitsschwellenwert größer als der erste Ähnlichkeitsschwellenwert ist, wobei das Auswählen das Auswählen des jeweiligen Vergleichsbildes ($I_{Comp}$) in jeder Kombination umfasst, für die bestimmt wurde, dass das Referenzbild ($I_{Ref}$) und das jeweilige Vergleichsbild ($I_{Comp}$) einen jeweiligen Ähnlichkeitsgrad aufweisen, wenn sie unter Verwendung des jeweiligen Versatzes in Bezug zueinander registriert werden, der größer als der erste Ähnlichkeitsschwellenwert und kleiner als der zweite Ähnlichkeitsschwellenwert ist.

9. Computerimplementiertes Verfahren nach Anspruch 7 oder Anspruch 8, wobei

der jeweilige Translationsversatz (t) zwischen dem Referenzbild ($I_{Ref}$) und dem jeweiligen Vergleichsbild ($I_{Comp}$) in jeder Kombination durch Berechnung einer Kreuzkorrelation unter Verwendung des Referenzbildes ($I_{Ref}$) und des jeweiligen Vergleichsbildes ($I_{Comp}$) bestimmt wird, und
der jeweilige Grad der Ähnlichkeit zwischen dem Referenzbild ($I_{Ref}$) und dem jeweiligen Vergleichsbild ($I_{Comp}$), wenn sie unter Verwendung des jeweiligen Translationsoffsets (t) zueinander registriert sind, durch Bestimmen eines Maximalwerts der berechneten Kreuzkorrelation bestimmt wird.

10. Computerimplementiertes Verfahren nach einem der vorhergehenden Ansprüche, das ferner das Erzeugen jedes Bildes der ersten Bildfolge (20) durch Segmentieren eines jeweiligen Bildes einer zweiten Bildfolge eines zweiten Bereichs der Retina (40) umfasst, beispielsweise so, dass das Bild des ersten Bereichs (30) ein Segment des jeweiligen Bildes der zweiten Bildfolge ist.

11. Computerimplementiertes Verfahren nach einem der vorhergehenden Ansprüche, wobei die erste Bildsequenz (20) eine Sequenz von Autofluoreszenzbildern des ersten Bereichs (30) der Netzhaut (40) des Auges (50) umfasst.

12. Computerimplementiertes Verfahren zum Trainieren eines maschinellen Lernalgorithmus zum Filtern von Rauschen aus Netzhautbildern, wobei das Verfahren umfasst

Erzeugen (S100) von Grundwahrheits-Trainings-Zieldaten durch Verarbeiten jeder Sequenz einer Vielzahl von Sequenzen von Netzhautbildern, um ein jeweiliges gemitteltes Netzhautbild zu erzeugen, wobei jedes gemittelte Netzhautbild gemäß dem computerimplementierten Verfahren nach einem der vorhergehenden Ansprüche erzeugt wird;
Erzeugen (S200) von Trainings-Eingangsdaten durch Auswählen eines jeweiligen Bildes aus jeder der Bildsequenzen; und
Verwenden (S300) der Grundwahrheits-Trainingszieldaten und der Trainingseingabedaten zum Trainieren des

**EP 4 575 980 B1**

maschinellen Lernalgorithmus zum Filtern von Rauschen aus Retinabildern.

13. Computerprogramm (345), das computerlesbare Anweisungen umfasst, die, wenn sie von mindestens einem Prozessor (320) ausgeführt werden, den mindestens einen Prozessor (320) veranlassen, ein Verfahren nach einem der vorhergehenden Ansprüche auszuführen.

14. Datenverarbeitungsvorrichtung (60), die so beschaffen ist, dass sie eine Folge von Bildern (20) eines Bereichs (30) der Netzhaut (40) eines Auges (50) verarbeitet, um ein gemitteltes Bild (70) des Bereichs (30) zu erzeugen, wobei die Datenverarbeitungsvorrichtung (60) mindestens einen Prozessor (320) und mindestens einen Speicher (340) umfasst, der computerlesbare Befehle speichert, die, wenn sie von dem mindestens einen Prozessor (320) ausgeführt werden, den mindestens einen Prozessor (320) dazu veranlassen

Bestimmen, für jede Kombination eines Referenzbildes ($I_{Ref}$), das aus der Sequenz von Bildern ausgewählt ist, und eines jeweiligen Vergleichsbildes ($I_{Comp}$), das ein Bild aus den verbleibenden Bildern in der Sequenz (20) ist, eines jeweiligen Translationsversatzes (t) zwischen dem Referenzbild ($I_{Ref}$) und dem jeweiligen Vergleichsbild ($I_{Comp}$) ;
Vergleichen der Größe jedes bestimmten Translationsversatzes (t) mit einem Translationsversatz-Schwellenwert (T), um zu bestimmen, ob der Translationsversatz (t) kleiner als der Translationsversatz-Schwellenwert (T) ist;
Auswählen des jeweiligen Vergleichsbildes ($I_{Comp}$) in jeder Kombination, für die der jeweilige Translationsversatz (t) zwischen dem Referenzbild ($I_{Ref}$) und dem jeweiligen Vergleichsbild ($I_{Comp}$) als kleiner als der Translationsversatz-Schwellenwert (T) bestimmt worden ist; und
Verwenden der ausgewählten Vergleichsbilder (25), um das gemittelte Bild (70) des Bereichs (30) zu erzeugen, indem:

Ausrichten der ausgewählten Vergleichsbilder (25) in Bezug aufeinander; und
Erzeugen des gemittelten Bildes (70) des ersten Bereichs (30) durch Mitteln der registrierten Bilder, derart, dass der jeweilige Pixelwert jedes Pixels in dem gemittelten Bild (70) ein Mittelwert der Pixelwerte von entsprechend angeordneten Pixeln in den registrierten Bildern ist, die einem jeweiligen gemeinsamen Ort in dem Bereich (30) der Retina (40) zugehören,

wobei der Translationsversatz-Schwellenwert (T) derart ist, dass, wenn die Folge von Bildern (20) mindestens ein Bild umfasst, das gegenüber dem Referenzbild ($I_{Ref}$) um einen Translationsversatz versetzt ist, der größer ist als der Translationsversatz-Schwellenwert (T), und Bilder, die von dem Referenzbild ($I_{Ref}$) um entsprechende Translationsversätze versetzt sind, die kleiner als der Translationsversatz-Schwellenwert (T) sind, das gemittelte Bild (70) mehr Textur zeigt, die eine Struktur in dem ersten Bereich (30) der Retina (40) angibt, als ein gemitteltes Referenzbild (75), das aus den Bildern in der Bildsequenz (20) erzeugt wird.

15. Ein ophthalmisches Abbildungssystem (100), das Folgendes umfasst:

eine ophthalmische Abbildungsvorrichtung (10), die so angeordnet ist, dass sie eine Folge von Bildern (20) eines Bereichs (30) der Netzhaut (40) eines Auges (50) erfasst; und
eine Datenverarbeitungsvorrichtung (60) nach Anspruch 14, die eingerichtet ist, die Folge von Bildern (20), die von der ophthalmischen Abbildungsvorrichtung (10) erfasst wird, zu verarbeiten, um ein gemitteltes Bild (70) des Bereichs (30) der Retina (40) zu erzeugen.

**Revendications**

1. Procédé mis en œuvre par ordinateur pour traiter une première séquence d'images (20) d'une première région (30) de la rétine (40) d'un œil (50) afin de générer une image moyenne (70) de la première région (30), le procédé comprenant :

la détermination (S10), pour chaque combinaison d'une image de référence ($I_{Ref}$) sélectionnée parmi la première séquence d'images (20) et d'une image de comparaison respective ($I_{Comp}$) qui est une image parmi des images restantes de la première séquence (20), d'un décalage de translation respectif (t) entre l'image de référence ($I_{Ref}$) et l'image de comparaison respective ($I_{Comp}$) ;
la comparaison (S20) de l'amplitude de chaque décalage de translation déterminé (t) à un seuil de décalage de translation (T) afin de déterminer si le décalage de translation (t) est inférieur au seuil de décalage de translation (T) ;

la sélection (S30) de l'image de comparaison respective (I$_{Comp}$) dans chaque combinaison pour laquelle le décalage de translation respectif (t) entre l'image de référence (I$_{Ref}$) et l'image de comparaison respective (I$_{Comp}$) a été déterminé comme étant inférieur au seuil de décalage de translation (T); et

l'utilisation (S40) des images de comparaison sélectionnées (25) pour générer l'image moyenne (70) de la première région (30), en :

enregistrant les images de comparaison sélectionnées (25) les unes par rapport aux autres ; et générant l'image moyenne (70) de la première région (30) en faisant la moyenne des images enregistrées, de telle sorte que la valeur de pixel respective de chaque pixel dans l'image moyenne (70) soit une moyenne des valeurs de pixel de pixels situés de manière correspondante dans les images enregistrées qui correspondent à un emplacement commun respectif dans la région (30) de la rétine (40),

dans lequel le seuil de décalage de translation (T) est tel que, lorsque la première séquence d'images (20) comprend au moins une image qui est décalée par rapport à l'image de référence (I$_{Ref}$) d'un décalage de translation supérieur au seuil de décalage de translation (T), et des images qui sont décalées par rapport à l'image de référence (I$_{Ref}$) de décalages de translation respectifs (t) qui sont inférieurs au seuil de décalage de translation (T), l'image moyenne (70) présente plus de texture indicative d'une structure dans la première région (30) de la rétine (40) qu'une image moyenne de référence (75) générée à partir des images de la première séquence d'images (20).

2. Procédé mis en œuvre par ordinateur selon la revendication 1, dans lequel le décalage de translation respectif (t) entre l'image de référence (I$_{Ref}$) et l'image de comparaison respective (I$_{Comp}$) dans chaque combinaison est déterminé (S10) par l'un des moyens suivants :

le calcul d'une corrélation croisée en utilisant l'image de référence (I$_{Ref}$) et l'image de comparaison respective (I$_{Comp}$) ; ou

le calcul d'une transformée de Fourier inverse d'un spectre de puissance croisée normalisé calculé en utilisant l'image de référence (I$_{Ref}$) et l'image de comparaison respective (I$_{Comp}$).

3. Procédé mis en œuvre par ordinateur selon la revendication 1 ou la revendication 2, comprenant en outre :

la détermination, pour chaque combinaison de l'image de référence (I$_{Ref}$) et de l'image de comparaison respective (I$_{Comp}$), d'un décalage de rotation respectif ($\Delta\varphi$) entre l'image de référence (I$_{Ref}$) et l'image de comparaison respective (I$_{Comp}$) ; et

la comparaison de l'amplitude de chaque décalage de rotation ($\Delta\varphi$) avec un seuil de décalage de rotation ($\Theta$) afin de déterminer si le décalage de rotation ($\Delta\varphi$) est inférieur au seuil de décalage de rotation ($\Theta$),

dans lequel la sélection (S30) comprend la sélection de l'image de comparaison respective (I$_{Comp}$) dans chaque combinaison pour laquelle le décalage de rotation respectif ($\Delta\varphi$) a été déterminé comme étant inférieur au seuil de décalage de rotation ($\Theta$).

4. Procédé mis en œuvre par ordinateur selon la revendication 3, dans lequel le décalage de rotation respectif ($\Delta\varphi$) entre l'image de référence (I$_{Ref}$) et l'image de comparaison respective (I$_{Comp}$) dans chaque combinaison est déterminé par l'un des moyens suivants :

le calcul de corrélations croisées en utilisant des versions pivotées de l'image de comparaison respective (I$_{Comp}$); ou

le calcul d'une transformée de Fourier inverse d'un spectre de puissance croisée normalisé calculé en utilisant des transformations de l'image de référence (I$_{Ref}$) et de l'image de comparaison respective (I$_{Comp}$) en coordonnées polaires.

5. Procédé mis en œuvre par ordinateur selon l'une quelconque des revendications précédentes, dans lequel les images de comparaison sélectionnées (25) sont enregistrées les unes par rapport aux autres en redistribuant des valeurs de pixels de l'une des images de la paire selon une transformation géométrique respective entre des systèmes de coordonnées des images de la paire.

6. Procédé mis en œuvre par ordinateur selon la revendication 5, dans lequel la transformation géométrique respective entre les systèmes de coordonnées d'image des images dans chaque paire d'images de comparaison sélectionnées (25) consiste en au moins l'un des éléments suivants :

une translation respective, d'un premier nombre entier de pixels, le long d'une première direction de matrice de pixels le long de laquelle des pixels dans les images de comparaison sélectionnées (25) sont disposés en matrice ; ou

une translation respective, d'un deuxième nombre entier de pixels, le long d'une deuxième direction de matrice de pixels le long de laquelle les pixels dans les images de comparaison sélectionnées (25) sont disposés en matrice, la deuxième direction de matrice de pixels étant orthogonale à la première direction de matrice de pixels.

7. Procédé mis en œuvre par ordinateur selon l'une quelconque des revendications précédentes, comprenant en outre :

la détermination (S15), pour chaque combinaison de l'image de référence ($I_{Ref}$) et d'une image de comparaison respective ($I_{Comp}$), d'un degré de similarité respectif entre l'image de référence ($I_{Ref}$) et l'image de comparaison respective ($I_{Comp}$) lorsqu'enregistrées l'une par rapport à l'autre en utilisant le décalage de translation respectif ; et

la comparaison (S25) de chaque degré de similarité déterminé à un premier seuil de similarité afin de déterminer si le degré de similarité déterminé est supérieur au premier seuil de similarité, dans lequel la sélection comprend la sélection (S30') de l'image de comparaison respective ($I_{Comp}$) dans chaque combinaison pour laquelle l'image de référence ($I_{Ref}$) et l'image de comparaison respective ($I_{Comp}$) ont été déterminées comme ayant un degré de similarité respectif, lorsqu' enregistrées l'une par rapport à l'autre en utilisant le décalage respectif, qui est supérieur au premier seuil de similarité.

8. Procédé mis en œuvre par ordinateur selon la revendication 7, comprenant en outre la comparaison de chaque degré de similarité déterminé avec un deuxième seuil de similarité afin de déterminer si le degré de similarité déterminé est inférieur au deuxième seuil de similarité, le deuxième seuil de similarité étant supérieur au premier seuil de similarité, dans lequel la sélection comprend la sélection de l'image de comparaison respective ($I_{Comp}$) dans chaque combinaison pour laquelle l'image de référence ($I_{Ref}$) et l'image de comparaison respective ($I_{Comp}$) ont été déterminées comme ayant un degré de similarité respectif, lorsqu'enregistrées l'une par rapport à l'autre en utilisant le décalage respectif, qui est supérieur au premier seuil de similarité et inférieur au deuxième seuil de similarité.

9. Procédé mis en œuvre par ordinateur selon la revendication 7 ou 8, dans lequel

le décalage de translation respectif (t) entre l'image de référence ($I_{Ref}$) et l'image de comparaison respective ($I_{Comp}$) dans chaque combinaison est déterminé en calculant une corrélation croisée en utilisant l'image de référence ($I_{Ref}$) et l'image de comparaison respective ($I_{Comp}$), et

le degré de similarité respectif entre l'image de référence ($I_{Ref}$) et l'image de comparaison respective ($I_{Comp}$), lorsqu'enregistrées l'une par rapport à l'autre en utilisant le décalage de translation respectif (t), est déterminé en déterminant une valeur maximale de la corrélation croisée calculée.

10. Procédé mis en œuvre par ordinateur selon l'une quelconque des revendications précédentes, comprenant en outre la génération de chaque image de la première séquence d'images (20) en segmentant une image respective d'une deuxième séquence d'images d'une deuxième région de la rétine (40), de telle sorte que l'image de la première région (30) soit un segment de l'image respective de la deuxième séquence d'images.

11. Procédé mis en œuvre par ordinateur selon l'une quelconque des revendications précédentes, dans lequel la première séquence d'images (20) comprend une séquence d'images d'autofluorescence de la première région (30) de la rétine (40) de l'œil (50).

12. Procédé mis en œuvre par ordinateur pour entraîner un algorithme d'apprentissage automatique à filtrer du bruit dans des images rétiniennes, le procédé comprenant :

la génération (S100) de données cibles d'apprentissage de référence en traitant chaque séquence d'une pluralité de séquences d'images rétiniennes afin de générer une image rétinienne moyenne respective, dans lequel chaque image rétinienne moyenne est générée conformément au procédé mis en œuvre par ordinateur de l'une quelconque des revendications précédentes ;

la génération (S200) de données d'entrée d'apprentissage en sélectionnant une image respective dans chacune des séquences d'images ; et

l'utilisation (S300) des données cibles d'apprentissage de référence et des données d'entrée d'apprentissage pour entraîner l'algorithme d'apprentissage automatique à filtrer du bruit dans les images rétiniennes.

13. Programme informatique (345) comprenant des instructions lisibles par ordinateur qui, lorsqu'elles sont exécutées par au moins un processeur (320), amènent ledit au moins un processeur (320) à exécuter un procédé selon l'une quelconque des revendications précédentes.

14. Appareil de traitement de données (60) agencé pour traiter une séquence d'images (20) d'une région (30) de la rétine (40) d'un œil (50) afin de générer une image moyenne (70) de la région (30), l'appareil de traitement de données (60) comprenant au moins un processeur (320) et au moins une mémoire (340) stockant des instructions lisibles par ordinateur qui, lorsqu'elles sont exécutées par le au moins un processeur (320), amènent le au moins un processeur (320) à :

déterminer, pour chaque combinaison d'une image de référence ($I_{Ref}$) sélectionnée parmi la séquence d'images et d'une image de comparaison respective ($I_{Comp}$) qui est une image parmi des images restantes de la séquence (20), un décalage de translation respectif (t) entre l'image de référence ($I_{Ref}$) et l'image de comparaison respective ($I_{Comp}$) ;
comparer la magnitude de chaque décalage de translation déterminé (t) avec un seuil de décalage de translation (T) afin de déterminer si le décalage de translation (t) est inférieur au seuil de décalage de translation (T) ;
sélectionner l'image de comparaison respective ($I_{Comp}$) dans chaque combinaison pour laquelle le décalage de translation respectif (t) entre l'image de référence ($I_{Ref}$) et l'image de comparaison respective ($I_{Comp}$) a été déterminé comme étant inférieur au seuil de décalage de translation (T); et
utiliser les images de comparaison sélectionnées (25) pour générer l'image moyenne (70) de la région (30), en :

enregistrant les images de comparaison sélectionnées (25) les unes par rapport aux autres ; et
générant l'image moyenne (70) de la première région (30) en faisant la moyenne des images enregistrées, de telle sorte que la valeur de pixel respective de chaque pixel dans l'image moyenne (70) soit une moyenne des valeurs de pixel de pixels situés de manière correspondante dans les images enregistrées qui correspondent à un emplacement commun respectif dans la région (30) de la rétine (40),

dans lequel le seuil de décalage de translation (T) est tel que, lorsque la séquence d'images (20) comprend au moins une image qui est décalée par rapport à l'image de référence ($I_{Ref}$) d'un décalage de translation supérieur au seuil de décalage de translation (T), et des images qui sont décalées par rapport à l'image de référence ($I_{Ref}$) de décalages de translation respectifs qui sont inférieurs au seuil de décalage de translation (T), l'image moyenne (70) présente plus de texture indicative d'une structure dans la première région (30) de la rétine (40) qu'une image moyenne de référence (75) générée à partir des images de la séquence d'images (20).

15. Système d'imagerie ophtalmique (100) comprenant :

un dispositif d'imagerie ophtalmique (10) agencé pour acquérir une séquence d'images (20) d'une région (30) de la rétine (40) d'un œil (50) ; et
un appareil de traitement de données (60) selon la revendication 14, qui est agencé pour traiter la séquence d'images (20) acquises par le dispositif d'imagerie ophtalmique (10) afin de générer une image moyenne (70) de la région (30) de la rétine (40).

Fig. 1

Fig. 2

Fig. 3

Determine, for each combination of a reference image selected from the first sequence of images and a respective comparison image being an image from remaining images in the first sequence, a respective offset between the reference image and the respective comparison image

S10

Compare each determined offset with an offset threshold to determine whether the offset is smaller than the offset threshold

S20

Select the respective comparison image in each combination for which the respective offset between the reference image and the respective comparison image has been determined to be smaller than the offset threshold

S30

Use the selected comparison images to generate the averaged image of the first region

S40

Fig. 4

Fig. 5

Fig. 6A

Fig. 6B

Fig. 6C

EP 4 575 980 B1

Fig. 7

Determine, for each combination of a reference image selected from the first sequence of images and a respective comparison image being an image from remaining images in the first sequence, a respective offset between the reference image and the respective comparison image    S10

Determine, for each combination of the reference image and a respective comparison image, a respective degree of similarity between the reference image and the respective comparison image when registered with respect to each other using the respective offset    S15

Compare each determined offset with an offset threshold to determine whether the offset is smaller than the offset threshold    S20

Compare each determined degree of similarity with a first similarity threshold to determine whether the determined degree of similarity is greater than the first similarity threshold    S25

Select the respective comparison image in each combination for which the respective offset is smaller than the offset threshold, and for which the respective degree of similarity is greater than the first similarity threshold    S30'

Use the selected comparison images to generate the averaged image of the first region    S40

Fig. 8

Fig. 9B

Fig. 9A

Generate ground truth training target data by processing each sequence of a plurality of sequences of retinal images to generate a respective averaged retinal image ⌠ S100

Generate training input data by selecting a respective image from each of the sequences of images ⌠ S200

Use the ground truth training target data and the training input data to train the a machine learning algorithm to filter noise from retinal images ⌠ S300

Fig. 10

Generate ground truth training target data by processing each sequence of a plurality of sequences of retinal images to generate a respective averaged retinal image ⌐ S100

Determine, for each combination of the reference image and a respective comparison image, a respective offset between the reference image and the respective comparison image ⌐ S110

Compare each determined offset with the offset threshold to determine whether the offset is smaller than the offset threshold ⌐ S120

Select the respective comparison image in each combination for which the respective offset between the reference image and the respective comparison image has been determined to be smaller than the offset threshold ⌐ S130

Use the selected comparison images to generate the respective averaged image of the region ⌐ S140

Return

Fig. 11

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 2019130170 A1
- CN 109345469 A

- EP 3668369 B1

### Non-patent literature cited in the description

- **J. LUCE et al.** Medical Image Registration Using the Fourier Transform. *International Journal of Medical Physics, Clinical Engineering and Radiation Oncology*, February 2014, vol. 3, 49-55

- **R. BERGMAN et al.** Detection of Textured Areas in Images Using a Disorganization Indicator Based on Component Counts. *J. Electronic Imaging.*, 2008, vol. 17, 043003
- **R. S. THAKUR et al.** Image De-Noising With Machine Learning: A Review. *IEEE Access*, 2021, vol. 9, 93338-93363